# EUROPÄISCHE PATENTSCHRIFT

(11) **EP 2 655 360 B1**
(45) Veröffentlichungstag und Bekanntmachung des Hinweises auf die Patenterteilung: **20.04.2016**
(21) Anmeldenummer: 11802377.9
(22) Anmeldetag: 19.12.2011
(51) Int. Cl.: C07D 403/04

(54) **VERFAHREN ZUR HERSTELLUNG VON N-SULFONYLSUBSTITUIERTEN OXINDOLEN**
METHOD FOR PRODUCING N-SULFONYL-SUBSTITUTED OXINDOLES
PROCÉDÉ DE PRÉPARATION D'OXINDOLES N-SULFONYLE SUBSTITUÉS

(30) Priorität: 21.12.2010 EP 10196205; 21.12.2010 US 201061425349 P; 25.03.2011 EP 11159875; 25.03.2011 US 201161467598 P
(43) Veröffentlichungstag der Anmeldung: 30.10.2013
(73) Patentinhaber: Bayer Intellectual Property GmbH, 40789 Monheim am Rhein (DE)
(72) Erfinder: KARIG, Gunter, 65719 Hofheim (DE); FORD, Mark James, 61389 Schmitten (DE); SIEGEL, Konrad, 40597 Düsseldorf (DE); SCHNATTERER, Stefan, 65795 Hattersheim (DE)
(74) Vertreter: BIP Patents
(86) Internationale Anmeldenummer: PCT/EP2011/073282
(87) Internationale Veröffentlichungsnummer: WO 2012/084854

(56) Entgegenhaltungen:
- US-A1- 2005 070 718
- US-A1- 2009 318 406
- US-A1- 2010 069 384

## Beschreibung

Die vorliegende Anmeldung betrifft ein Verfahren zur selektiven N-Sulfonylierung von Oxindolen, insbesondere ein Verfahren zur N-Sulfonylierung von 3-Triazinyloxindolen, sowie deren Verwendung als Zwischenprodukte für die Synthese von Feinchemikalien und von Wirkstoffen im Bereich der Landwirtschaft.

Außerdem betrifft die vorliegende Anmeldung N-sulfonylsubstituierte 3-Triazinyloxindole sowie deren Verwendung als Zwischenprodukte für die Synthese von Feinchemikalien im Bereich der Landwirtschaft.

Die aus dem Stand der Technik bekannten Verfahren zur N-Sulfonylierung von Oxindolen haben häufig nicht die Durchführung der Reaktion im technischen, d.h. im industriellen, Maßstab zum Ziel. Dementsprechend ist die Verwendung der bei bislang bekannten Verfahren eingesetzten Aktivierungsmittel, insbesondere der zur Deprotonierung eingesetzten Basen, nicht für die Durchführung der Reaktion im industriellen Maßstab geeignet.

Außerdem offenbart der Stand der Technik kein allgemeines Prinzip zur möglichst selektiven Herstellung von N-sulfonylsubstituierten Oxindolen. Es ist bekannt, dass Reaktionen zur Sulfonylierung von Oxindolen abhängig von den Reaktionsbedingungen unselektiv verlaufen können, wobei (anstatt des gewünschten N-sulfonylierten Oxindols) auch ein O-sulfonyliertes Produkt oder ein zweifach, d.h. am Sauerstoff und am Stickstoff, sulfonyliertes Produkt gebildet werden kann.

Beispiele zum Beleg der geringeren Selektivität der Sulfonylierung von Oxindolen finden sich z.B. in Synthetic Commun. (1992) 22, 2987 oder Org. Biomol. Chem. (2009) 7, 3413. Dabei ist davon auszugehen, dass die Wahrscheinlichkeit zur Bildung von O-sulfonylierten Produkten besonders bei Oxindolen, bei denen mindestens einer der Substituenten in 3-Position Wasserstoff ist, erhöht ist.

### Deprotonierung

Die der Sulfonylierung vorausgehende Deprotonierung des Oxindols in 1-Postition ist vermutlich eine wichtige Voraussetzung für die Selektivität der Sulfonylierung. Die Substitution des Oxindol-Gerüsts im Übrigen, vor allem aber die Gegenwart einer als Aktivierungsmittel dienenden Base, können für den Verlauf der Sulfonylierung ebenfalls entscheidend sein.

Es ist bekannt, dass Wasserstoff an Heteroatomen in aliphatischen, aromatischen, oder heteroaromatischen Verbindungen gegen funktionelle Substituenten, wie z.B. eine Sulfonylgruppe ausgetauscht werden kann. Ebenso ist bekannt, dass N-unsubstituierte oder N-monosubstituierte Amide mit Sulfonylierungsreagenzien wie Sulfonylchloriden in Gegenwart von Basen zu N-Sulfonylamiden reagieren.

Während Amidsulfonylierungen auch mit schwachen Basen, wie z.B. Pyridin oder Triethylamin, durchgeführt werden können, ist allgemein bekannt (Blakemore, P. R.: N-Sulfonylation of Amides, Science of Synthesis, 21 (2005), p.879), dass die Umsetzung in den meisten Fällen besser gelingt, wenn die Deprotonierung des Substrates mit starken Basen wie z.B. Natriumhydrid, Butyllithium oder Lithiumhexamethyldisilazan durchgeführt wird, wobei die Deprotonierung der Zugabe des Sulfonylierungsreagenzes zu dem sich aufgrund der Deprotonierung ergebenden nukleophilen Amidanion vorausgeht.

### N-sulfonylsubstituierte Oxindole

Verbindungen, die auf die im vorhergehenden Absatz genannte Weise hergestellt werden können, sind z.B. optional substituierte Oxindole (1,3-Dihydro-2H-indol-2-one), bei denen in 1-Position Wasserstoff gegen einen Sulfonylsubstituenten ausgetauscht wurde. Diese Verbindungen werden als N-sulfonylsubstituierte Oxindole bezeichnet.

### Phenylsubstituierte Oxindole

Eine Untergruppe der N-sulfonylsubstituierten Oxindole sind jene Oxindole, welche in 3-Position einen Phenylsubstituenten tragen. Beispiele für Synthesen von solchen Verbindungen, bei denen in 1-Position Wasserstoff gegen einen Sulfonylsubstituenten ausgetauscht wurde, finden sich z.B. in FR 2714378, US 1997/5594023, US 2004/180878, US 2005/70718, WO 2006/110917, WO 2006/072458, WO 2006/100080, WO 2006/100081, WO 2006/100082, WO 2008/107399, WO 2008/025735, US 2008/318923, US 2009/318406 sowie in Bioorg. Med. Chem. Lett. (1998), 8, 175; Chem. Commun. (2009) 26, 3955.

Gemeinsam ist den im vorgenannten Stand der Technik offenbarten Reaktionen, dass zur Sulfonylierung starke Basen, wie Natriumhydrid oder Kaliumtert-butylat, verwendet werden. Jedoch sind diese starken Basen empfindlich gegen Wasser und lassen sich daher nach einer wässrigen Aufarbeitung nicht unzersetzt wiedergewinnen. Darüber hinaus verursachen diese starken Basen nachteiligerweise die Entstehung von äquimolaren Mengen elementaren Wasserstoffs oder sind teuer. Daher ist die technische Anwendung dieser Basen nicht vorteilhaft.

Für die Verwendung der schwachen Base Triethylamin zur Sulfonylierung eines Oxindols, welches in 3-Position mit Phenyl sowie Imidazol-1-yl substituiert ist, finden sich im Stand der Technik kaum Beispiele (European Journal of Medicinal Chemistry (1981), 16, 373). Die chemische Ausbeute der Sulfonylierungsreaktion aus der vorgenannten Publikation beträgt allerdings nur 12% und ist daher für die Anwendung im technischen Maßstab nicht geeignet (Beispiel 1, Variante F).

### Hetarylsubstituierte Oxindole

Oxindole, welche in 3-Position einen Hetarylsubstituenten tragen, bilden eine weitere Untergruppe der N-sulfonylsubstituierten Oxindole. Beispiele für Reaktionen zur Gewinnung von N-sulfonylsubstituierten Oxindolen mit einem 6-Ring-Hetarylsubstituenten in 3-Position, bei denen Wasserstoff in 1-Position gegen einen Sulfonylsubstituenten ausgetauscht wird, sind 3-(3-pyridyl)-substituierte Oxindole (US 2005/70718, WO 2009/083559, WO 2008/80970), bzw. 3-(3,5-pyrimidyl)-substituierte Oxindole (US 2005/70718). Jedoch ist auch den im vorgenannten Stand der Technik offenbarten Reaktionen gemeinsam, dass zur Sulfonylierung starke Basen wie Natriumhydrid oder Kaliumtert-butylat verwendet werden, deren Anwendung im technischen Maßstab die oben beschriebenen Nachteile hat.

### N-Sulfonylierung von phenylsubstituierten Oxindolen mit Natriumcarbonat

In Schema 1 ist ein bekanntes Verfahren (J. Chem. Soc. (1957), 4789 - 4798) zur N-Sulfonylierung eines Oxindols, welches als Substituenten einen Phenylring in 3-Position trägt, zusammengefasst. Dieses Verfahren ist dadurch gekennzeichnet, dass es nicht wie in den im vorgenannten Stand der Technik offenbarten Reaktionen unter Verwendung von starken Basen wie Natriumhydrid oder Kaliumtert-butylat, sondern mit Natriumcarbonat als Base in Wasser/Aceton, durchgeführt wird. Für die beschriebene Umsetzung von 3-Phenyloxindol A mit 4-Methylbenzolsulfonylchlorid ist als Produkt B 3-Phenyl-1-toluol-p-sulphonyloxindol angegeben und eine Ausbeute von 41% genannt.

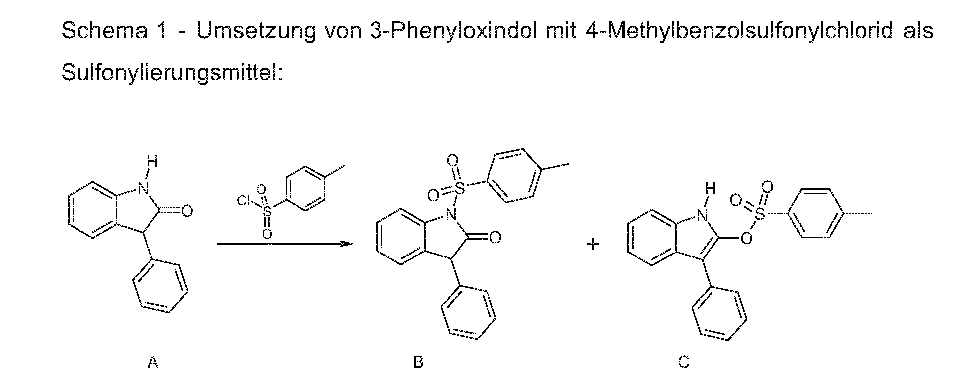

Zu Vergleichszwecken wurde die Reaktion unter den in J. Chem. Soc. (1957), 4789 - 4798, auf Seite 4796 beschriebenen Bedingungen zur Umsetzung von 3-Phenyloxindol nachgearbeitet, wobei als Sulfonylierungsmittel 4-Methylbenzolsulfonylchlorid und als Base Natriumcarbonat in Wasser/Aceton eingesetzt wurde. Nach Produktisolierung wurde durch NMR- Analytik festgestellt, dass bei dieser Reaktion nicht wie postuliert das N-sulfonylierte Produkt B als Hauptkomponente gebildet wird, sondern das O-sulfonylierte Produkt C (Beispiel 11). Somit ist das aus J. Chem. Soc. (1957), 4789 bekannte Verfahren zur Herstellung von N-sulfonylsubstituierten 3-(Phenyl)oxindolen nicht geeignet.

### N-sulfonylsubstituierten 3-Triazinyloxindole

N-sulfonylsubstituierte 3-Triazinyloxindole, die eine weitere Untergruppe der in 3-Position hetarylsubstituierten N-sulfonylsubstituierten Oxindole bilden, sind im Stand der Technik nicht beschrieben. Das gilt auch für Verfahren zur Herstellung von N-sulfonylsubstituierten 3-Triazinyloxindolen.

Eine in wirtschaftlicher Hinsicht besonders bedeutsame Gruppe der N-sulfonylsubstituierten 3-Triazinyloxindole sind jene Verbindungen, welche als Substituenten der N-Sulfonylgruppe ganz oder teilweise mit Fluor substituierte C₁- C₆ Alkylgruppen, insbesondere Difluormethyl und Trifluormethyl, oder (C₃-C₇)-Cycloalkylgruppen tragen. Diese Verbindungen eignen sich als Wirkstoffe in der Pharmazie oder der Landwirtschaft oder als Zwischenprodukte zur Herstellung von Feinchemikalien und Wirkstoffen im Bereich der Pharmazie oder der Landwirtschaft.

Wiederum zu Vergleichszwecken wurden die in J. Chem. Soc. (1957), 4789 - 4798, auf Seite 4796 beschriebenen Bedingungen auch auf ein 3-(Triazinyl)oxindol bei der Reaktion mit 4-Methylbenzolsulfonylchlorid angewendet. Jedoch konnte durch UV-Absorption und NMR-Analytik nachgewiesen werden, dass ebenfalls nicht das N-sulfonylierte Produkt, sondern das O-sulfonylierte Produkt gebildet wird (Beispiel 13). Somit ist das beschriebene Verfahren ebenfalls nicht geeignet, N-sulfonylsubstituierte 3-Triazinyloxindole herzustellen.

### N-Sulfonylierung von phenyl-substituierten Oxindolen mit Natriumhydrid

In US 2009/0318406 ist ein Verfahren zur Sulfonylierung in 1-Position eines Oxindols, welches als Substituenten in 3-Position einen substituierten Phenylring oder einen substituierten Piperazinring trägt, offenbart. Das Verfahren wird unter Verwendung von Natriumhydrid als Base in Tetrahydrofuran als Lösungsmittel bei 0°C durchgeführt.

Wiederum zu Vergleichszwecken wurden die in US 2009/0318406 auf Seite 19 Absatz 243 beschriebenen Bedingungen auch auf die Reaktion eines 3-Triazinyloxindols mit Difluormethansulfonylchlorid angewendet. Jedoch wurde festgestellt, dass nach 12 Stunden Reaktionszeit nahezu kein Umsatz zum gewünschten Produkt stattgefunden hatte (Beispiel 1, Variante D). Somit ist das in US 2009/0318406 beschriebene Verfahren nicht geeignet, N-sulfonylsubstituierte 3-Triazinyloxindole im technischen Maßstab herzustellen, zumindest wenn Difluormethansulfonylchlorid als Sulfonylierungsmittel verwendet wird.

### N-Sulfonylierung von phenyl-substituierten Oxindolen mit Kalium-tert.-butylat

In US 2010/69384 ist ein Verfahren zur Sulfonylierung in 1-Position eines Oxindols, welches als Substituenten in 3-Position einen substituierten Phenylring und eine Methylgruppe trägt, offenbart. Das Verfahren wird unter Verwendung von Kaliumtert.-butylat als Base in Tetrahydrofuran als Lösungsmittel bei -30°C durchgeführt.

Wiederum zu Vergleichszwecken wurden die in US 2010/69384 auf Seite 15 Beispiel 7A beschriebenen Bedingungen auch auf die Reaktion eines 3-(Triazinyl)oxindols mit Difluormethansulfonylchlorid angewendet. Jedoch wurde festgestellt, dass nach 12 Stunden Reaktionszeit nahezu kein Umsatz zum gewünschten Produkt stattgefunden hatte (Beispiel 1, Variante E). Somit ist auch das in US 2010/69384 beschriebene Verfahren nicht geeignet, N-sulfonylsubstituierte 3-Triazinyloxindole herzustellen, zumindest wenn Difluormethansulfonylchlorid als Sulfonylierungsmittel verwendet wird.

Auf das in European Journal of Medicinal Chemistry (1981), 16, 373 offenbarte Verfahren zur Sulfonylierung eines in 3-Position mit Phenyl sowie Imidazol-1-yl substituierten Oxindols wurde bereits eingegangen. Das Verfahren ist durch die Verwendung von Triethylamin als Base mit Dichlormethan als Lösungsmittel gekennzeichnet.

Wiederum zu Vergleichszwecken wurden die in Journal of Medicinal Chemistry (1981), 16, 373 beschriebenen Bedingungen auch auf die Reaktion eines 3-(Triazinyl)oxindols mit Difluormethansulfonylchlorid angewendet. Jedoch wurde festgestellt, dass nach 3 Stunden Reaktionszeit und Stehenlassen für 15 Stunden nahezu kein Umsatz zum gewünschten Produkt stattgefunden hatte (Beispiel 1, Variante F). Somit ist das beschriebene Verfahren nicht geeignet, N-sulfonylsubstituierte 3-Triazinyloxindole herzustellen, zumindest wenn Difluormethansulfonylchlorid als Sulfonylierungsmittel verwendet wird.

Die Aufgabe der Erfindung besteht vor diesem Hintergrund in der Bereitstellung eines möglichst selektiven Verfahrens zur Gewinnung von N-sulfonylsubstituierten Oxindolen, die in 3-Position einen sechsgliedrigen Heteroarylrest tragen, in technischem Maßstab.

Überraschend wurde nun gefunden, dass sich N-sulfonylsubstituierte 3-Triazinyloxindole (3-1) durch Austausch eines Wasserstoffatoms in 1-Position eines wahlweise substituierten 3-Triazinyloxindols (1) mit einer Sulfonylverbindung (2), welche eine geeignete Abgangsgruppe X trägt, in Gegenwart von in 1-Position substituierten Imidazolen oder einer Mischung aus in 1-Position substituierten Imidazolen mit tertiären Aminbasen oder einer Mischung aus in 1-Position substituierten Imidazolen mit substituierten Pyridinbasen, mit guter bis sehr guter Selektivität herstellen lassen (Schema 2).

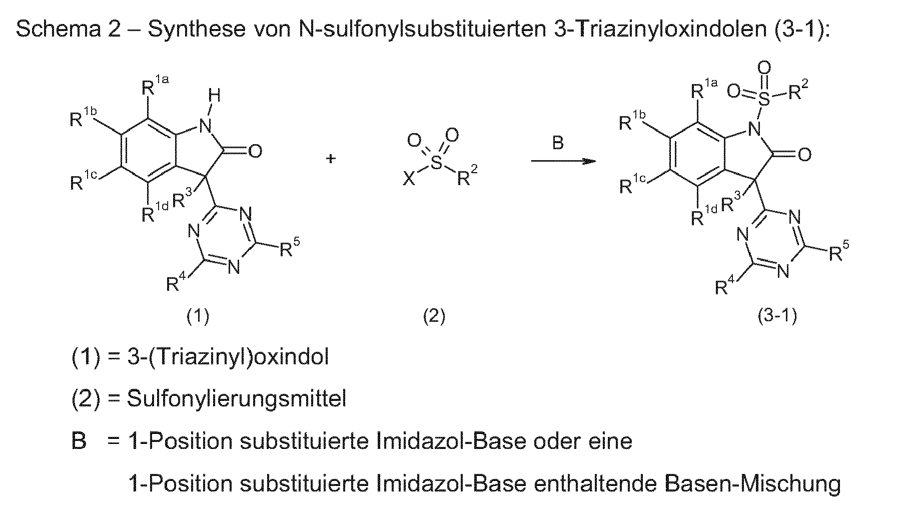

Imidazole haben in ihrer Wirkung als Basen den Vorteil, dass sie für den Einsatz im technischen Maßstab geeignet sind, weil sie insbesondere in Gegenwart von Wasser nicht zersetzlich sind und außerdem keine äquimolaren Mengen Wasserstoff erzeugen.

Somit wird die Aufgabe gelöst durch ein Verfahren zur Herstellung von N-sulfonylsubstituierten Oxindolen der Formel (3), die in 3-Position einen 6-Ring-Heteroarylsubsitutenten (Q) aufweisen, 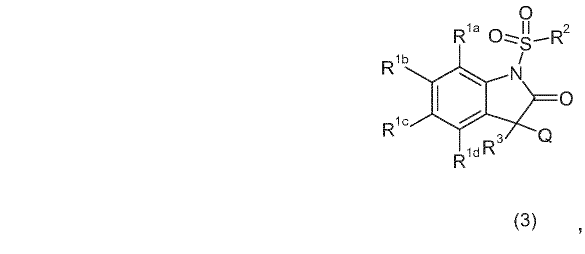 worin
R^{1a} bis R^{1d} unabhängig voneinander ausgewählt sind aus der Gruppe bestehend aus
Wasserstoff, Fluor, Chlor, Brom, Iod sowie aus
(C₁₋C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
      steht
R³ für
   Wasserstoff, oder
   (C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, steht, und
   Q für
      einen sechsgliedrigen heteroaromatischen Ring mit 1 bis 3 Stickstoffatomen steht, wobei der heteroaromatische Ring unsubstituiert ist oder durch einen oder mehrere Substituenten, ausgewählt aus der aus (C₁-C₄)-Alkyl, (C₁-C₄)-Alkoxy oder (C₃-C₇)-Cycloalkyl oder (C₃-C₇)-Cycloalkoxy oder (C₁-C₄)-Alkylthio bestehenden Gruppe, substituiert ist,
durch Umsetzung einer Oxindolverbindung der Formel (1) 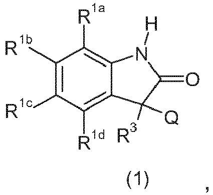 worin
R^{1a} bis R^{1d} sowie R³ und Q wie in Formel (3) definiert sind,
in einem Lösungsmittel mit einer Sulfonylverbindung (2) 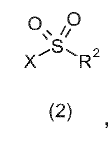 worin
R² wie in Formel (3) definiert ist, und
X als Abgangsgruppe für Fluor, Chlor, Brom, 1-Imidazolyl, 1 H-Benzotriazolyloxy, 1 H-Benzotriazolyl oder O-SO₂-R⁷, wobei R⁷ wie R² definiert ist und R² und R⁷ gleich oder verschieden sind, oder N(R⁸)SO₂R⁹, wobei R⁸ für Carbonyl und R⁹ für unsubstituiertes oder substituiertes Phenyl stehen und R⁸ und R⁹ miteinander verbunden sind oder nicht verbunden sind, steht,
wobei die Umsetzung in Gegenwart
- einer in 1-Position substituierten Imidazol-Base, oder
- eines Basen-Gemischs, das mindestens eine in 1-Position substituierte Imidazol-Base enthält,
durchgeführt wird.

Die in der Formel (1) unter die Definition von Q fallenden Heteroaryle umfassen insbesondere 1,3,5 Triazin-2-yl.

Charakterisiert werden die N-sulfonylsubstituierten 3-Triazinyloxindole sowie die im erfindungsgemäßen Verfahren eingesetzten 3-Triazinyloxindole neben dem Triazinrest jeweils durch den Rest R², welcher für die Verbindungen der vorliegenden Erfindung ein einheitliches Merkmal darstellt.

Die eingesetzten Basen zersetzen sich in Gegenwart von Wasser nicht und setzen während der Reaktion keinen Wasserstoff (H₂) frei, so wie dies zum Beispiel bei Natriumhydrid der Fall ist.

### Umwandlung

Zudem ist es überraschend, dass gerade in 1-Position substituierte Imidazol-Basen die Selektivität der N-Sulfonylierung gegenüber der O-Sulfonylierung von Oxindolen, die in 3-Position einen sechsgliedrigen Heteroarylrest tragen, deutlich verbessern. Das gilt besonders für die Gewinnung von N-sulfonylsubstituierten 3-Triazinyloxindolen.

Nachweislich besteht ein besonderer Vorteil der Verwendung von Imidazol-Basen im Zusammenhang mit dem vorliegenden Verfahren darin, dass die Basen die vollständige oder teilweise Umwandlung eines im Laufe der Reaktion als Haupt- oder Nebenkomponente gebildeten O-sulfonylierten hetarylsubstituierten Oxindols (D) oder eines anderen Intermediates in das gewünschte entsprechende N-sulfonylierte Oxindol (C) katalysieren können.

Veranschaulicht wird die Umwandlung für ein 3-Triazinyloxindol durch Schema 3 und die Tabellen 1 und 2 sowie beispielhaft belegt durch Beispiel 1 Variante B.

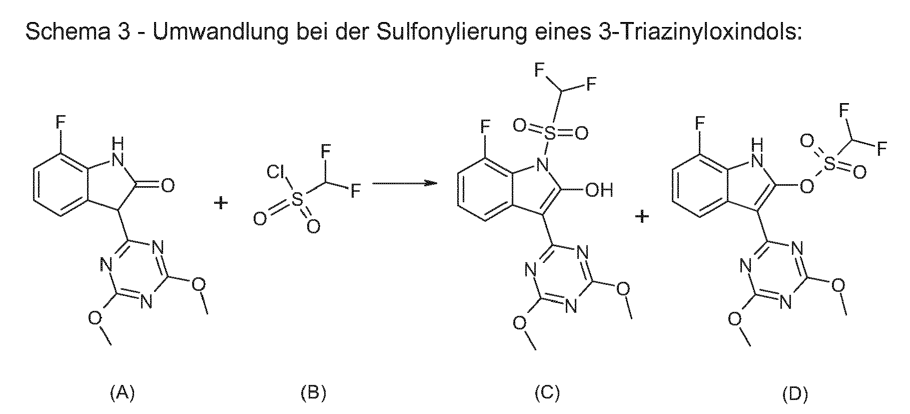

Die Umwandlung eines gebildeten O-sulfonylierten 3-Triazinyloxindols (D) in das gewünschte N-sulfonylierte 3-Triazinyloxindol (C) erfolgt entweder - direkt, d.h. das gewünschte N-sulfonylierte 3-Triazinyloxindol (C) wird direkt gebildet (Beispiel 1 Variante B), oder
- indirekt, d.h. nach Rückbildung des Edukts (A) wird das gewünschte N-sulfonylierte 3-Triazinyloxindol (C) gebildet.

Zum Nachweis der Umwandlung eines O-sulfonylierten 3-Triazinyloxindols (D) in das gewünschte N-sulfonylierte 3-Triazinyloxindol (C) wird zunächst ein aus 62 % O-sulfonyliertem 3-Triazinyloxindol (D) und aus 30 % N-sulfonyliertem 3-Triazinyloxindol (C) bestehendes Gemisch isoliert. (Die Anteile der Verbindungen A, C, D am Gesamtgemisch werden mittels HPLC festgestellt.)

Die eine Hälfte des isolierten Gemischs wird in Abwesenheit des Sulfonylierungsreagenzes Difluormethansulfonylchlorid (B) mit der Imidazol-Base 1-Methyl-1 H-Imidazol umgesetzt (siehe Tabelle 1).

Zum Vergleich wird die zweite Hälfte des isolierten Gemischs aus 62 % O-sulfonyliertem 3-Triazinyloxindol (D) und aus 30 % N-sulfonyliertem 3-Triazinyloxindol (C) ohne 1-Methyl-1-H-Imidazol und in Abwesenheit des Sulfonylierungsreagenzes Difluormethansulfonylchlorid (B) bei 0°C gerührt (siehe Tabelle 2).

**Tabelle 1 (nach Zugabe von 1-Methyl-1H-Imidazol)**

| HPLC nach | A | C | D | T° |
|---|---|---|---|---|
| 0 min | 1 % | 30 % | 62 % | 0°C |
| 1 h | 5 % | 27 % | 58 % | 0°C |
| 3h | 9 % | 31 % | 52 % | 0°C |
| 7h | 18 % | 37 % | 38 % | 0°C |
| über Nacht | 22 % | 58 % | 12 % | RT |

Die in Tabelle 1 zusammengefassten Ergebnisse zeigen, dass in Gegenwart von 1-Methyl-1 H-Imidazol eine Umwandlung von O-sulfonyliertem 3-Triazinyloxindol (D) in das gewünschte N-sulfonylierte 3-Triazinyloxindol (C) erfolgt.

Der Gehalt an dem gewünschten N-sulfonylierten 3-Triazinyloxindol (C) nimmt ausgehend von 30 % auf 58 % zu, d.h. die Imidazol katalysierte Umwandlung führt fast zu einer Verdoppelung an N-sulfonyliertem 3-Triazinyloxindol (C).

**Tabelle 2 (ohne Zugabe von 1-Methyl-1H-Imidazol)**

| HPLC nach | A | C | D | T° |
|---|---|---|---|---|
| 0 min | 1 % | 30 % | 62 % | 0°C |
| 1 h | 1 % | 27 % | 65 % | 0°C |
| 3 h | 2 % | 31 % | 61 % | 0°C |
| 7 h | 1 % | 27 % | 65 % | 0°C |
| über Nacht | 2 % | 30 % | 62 % | RT |

Die in Tabelle 2 zusammengefassten Ergebnisse zeigen, dass die gewünschte Umwandlung von O-sulfonyliertem 3-Triazinyloxindol (D) in das gewünschte N-sulfonylierte 3-Triazinyloxindol (C) ohne Zugabe von 1-Methyl-1 H-Imidazol nicht erfolgt.

Im Hinblick auf die erfindungsgemäßen Verbindungen werden weitere vorstehend und weiter unten verwendeten Bezeichnungen zusammenfassend erläutert. Diese sind dem Fachmann geläufig und haben insbesondere die im Folgenden erläuterten Bedeutungen:

Soweit in dieser Anmeldung auf ein "3-Triazinyloxindol" verwiesen wird, ist eine der von der allgemeinen Formel (1-1) umfassten Verbindungen gemeint.

Die bevorzugt als Edukte eingesetzten 3-Triazinyloxindole (1-1) 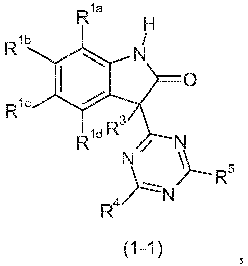 worin die Reste R^{1a} bis R^{1d}, R³, R⁴ und R⁵ wie oben definiert sind, können unter Anwendung der dem Fachmann bekannten Verfahren hergestellt werden.

Soweit in dieser Anmeldung auf eine "Sulfonylverbindung" verwiesen wird, ist eine der von der allgemeinen Formel (2) 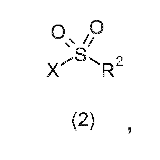 umfassten Verbindungen gemeint. In Verbindungen der Formel (2) sind R² und X wie oben definiert. Die als Edukte eingesetzten Sulfonylverbindungen (2) sind bekannt oder können unter Anwendung von dem Fachmann bekannten Verfahren hergestellt werden.

Soweit in dieser Anmeldung auf eine "tertäre Aminbase" verwiesen wird, ist eine der von der allgemeinen Formel (4) 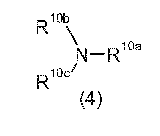 umfassten Verbindungen gemeint, wobei
R^{10a}, R^{10b} und R^{10c} unabhängig voneinander für verzweigtes oder unverzweigtes (C₁-C₆)-Alkyl stehen und mindestens einer der Substituenten R^{10a}, R^{10b} und R^{10c} in einer nicht-endständigen Position ein weiteres Heteroatom wie Sauerstoff oder Schwefel oder die Gruppe NR^{10d}, wobei R^{10d} für (C₁-C₆)-Alkyl steht, enthält, oder
R^{10b} und R^{10c} miteinander zu einem drei bis zehngliedrigen Ring verbunden sind und der Ring mindestens ein weiteres Heteroatome wie Sauerstoff oder Schwefel oder die Gruppe NR^{10d}, wobei R^{10d} für (C₁-C₆)-Alkyl steht, enthalten kann, oder
R^{10a}, R^{10b} und R^{10c} zusammen einen Bicyclus bilden, wobei das in Formel (4) enthaltene Stickstoffatom das eine Brückenkopfatom bildet und das andere Brückenkopfatom Kohlenstoff oder Stickstoff sein kann und im Falle, dass das andere Brückenkopfatom Kohlenstoff ist, einer oder beide Ringe noch mindestens ein weiteres Heteroatom wie Sauerstoff oder Schwefel oder die Gruppe NR^{10d}, wobei R^{10d} für (C₁-C₆)-Alkyl steht, enthalten und Ringatome auch über eine Doppelbindung verbunden sein können.

Wenn eine Verbindung der allgemeinen Formel (4) mehr als ein Heteroatom enthält, dann können die Heteroatome nicht direkt nebeneinander stehen.

Soweit in dieser Anmeldung auf eine "substituierte Pyridinbase" verwiesen wird, ist eine der von der allgemeinen Formel (5) 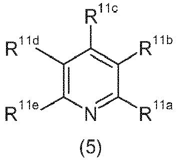 umfassten Verbindungen gemeint, wobei
R^{11a}, R^{11b}, R^{11c}, R^{11d} und R^{11e} unabhängig voneinander für Wasserstoff, Methyl, Ethyl, Propyl oder Isopropyl stehen und mindestens einer der Substituenten R^{11a}, R^{11b}, R^{11c}, R^{11d} und R^{11e} nicht Wasserstoff ist.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Alkyl bedeutet einen geradkettigen oder verzweigten offenkettigen, gesättigten Kohlenwasserstoffrest.

Der Ausdruck "(C₁-C₄)Alkyl" bedeutet eine Kurzschreibweise für Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind.
Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Aryl bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist.

Von der Systematik her ist Aryl in der Regel auch von dem Begriff "gegebenenfalls substituiertes Phenyl" umfasst.

Alkoxy bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Alkylthio bedeutet ein über ein Schwefelatom gebundenen Alkylrest, Alkenylthio bedeutet ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃,; Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Substituierte Reste, wie ein substituierter Alkyl-, Cycloalkyl-, Cycloalkenyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfinylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthioalkyl, Alkoxyalkyl, gegebenfalls substituiertes Mono- und Dialkylaminoalkyl und Hydroxyalkyl bedeuten.

Im Begriff "substituierte Reste", wie substituiertes Alkyl etc., sind als Substituenten neben den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und Dialkenylaminocarbonyl, Mono- und Dialkinylaminocarbonyl, Mono- und Dialkenylamino, Mono- und Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring sind auch cyclische Systeme mit jenen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Die jeweils unsubstitutierten oder substituierten Reste können verzweigt und unverzweigt sein. So umfasst zum Beispiel ein mit "C₄-Alkyl" bezeichneter Rest neben dem unverzweigten Butyl-Rest alle weiteren C₄-Isomere darunter auch *tert-*Butyl.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

In einer bevorzugten Ausführungsform steht R² für (C₁-C₆)-Alkyl oder (C₃-C₇)-Cycloalkyl, wobei der Alkylrest oder der Cycloalkylrest ganz oder teilweise mit Fluor substituiert ist und wobei Verbindungen der Formel (3-1), in denen
- R^{1a} für Fluor und
- R² für 2,2,-Difluorethyl oder 1,1,1-Trifluorethyl steht,
ausgenommen sind, weil die Bildung dieser spezifischen Verbindungen unter den in dieser Anmeldung beschriebenen Reaktionsbedingungen nicht nachgewiesen werden konnte. Dies schließt jedoch nicht aus, dass diese Verbindungen, z.B. durch Variation der Zusammensetzung des Basengemischs oder aber durch Variation der Reaktionstemperatur oder des während der Reaktion herrschenden Drucks, hergestellt werden können.

In der vorgenannten bevorzugten Ausführungsform stehen
R⁴ und R⁵ unabhängig voneinander jeweils für
Wasserstoff,
(C₁-Cₑ)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist.

In einer weiteren bevorzugten Ausführungsform ist R³ = H. In diesem Fall können Verbindungen der Formel (3-1) ganz oder teilweise auch in der tautomeren Enolform oder als Salze (3-1 a) 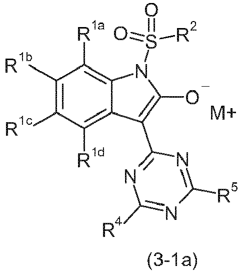 vorliegen, worin
R^{1a} bis R^{1d} sowie R², R⁴ und R⁵ wie oben definiert sind und
M für Li, Na, K, Cs, Ba, Mg, Ca, Zn oder N(R^{c})₄ mit R^{c} = H oder C₁-C₆ Alkyl, steht und die Anzahl der Gegenionen M⁺ sich nach der jeweiligen Ladung richtet, so dass die Verbindung der allgemeinen Formel (3-1a) insgesamt neutral ist.

In einer am meisten bevorzugten Ausführungsform wird
Difluormethansulfonylchlorid als Sulfonylierungsmittel verwendet, d.h. R² steht für Difluormethyl.

Bei den erfindungsgemäß eingesetzten Imidazolbasen handelt es sich bevorzugt um
- 1-(C₁-C₆)-Alkyl-1H-Imidazol,
- 1-(C₁-C₇)-Cycloalkyl-1H-Imidazol,
- 1-Benzyl-1 H-Imidazol,
- 1-Aryl-1 H-Imidazol,
- 1-Hetaryl-1 H-Imidazol, oder
- ein Gemisch, das mindestens zwei der genannten in 1-Position substituierten Imidazol-Basen enthält.

Besonders bevorzugt sind die Imidazol-Basen 1-Methyl-1 H-Imidazol, 1-Butyl-1H-Imidazol oder 1-Benzyl-1 H-Imidazol, die einzeln oder im Gemisch eingesetzt werden können, wobei die Verwendung von 1-Methyl-1 H-Imidazol ganz besonders bevorzugt ist.

Diese Basen haben gegenüber anderen Basen, wie z.B. den im Stand der Technik beschriebenen Basen Natriumhydrid oder Kaliumtert-butylat, den Vorteil, dass sie keine äquimolaren Mengen Wasserstoff erzeugen oder in Kontakt mit Wasser nicht zersetzt werden und somit für den Einsatz im technischen (industriellen) Maßstab geeignet sind.

Tertiäre Aminbasen oder substituierte Pyridinbasen alleine führen nicht zu einer N-Sulfonylierung des Eduktes. Es konnte beispielsweise gezeigt werden, dass bei der Sulfonylierung unter Verwendung von Trifluormethansulfonsäureanhydrid mit 1-Methyl-1 H-Imidazol als Base das N-sulfonylierte Produkt gebildet wird (Beispiel 9), während die Verwendung von 1,4-Diazabicyclo[2.2.2]octan (DABCO) als Base bevorzugt zum O-sulfonylierte Produkt führt (Beispiel 10). Die Unterscheidung der N- bzw. O-Sulfonylierung kann durch die Bestimmung der UV-Absorption der Produkte erfolgen. Die N-sulfonylierten Produkte zeigen ein Absorptions-Maximum bei 360 nm, welches die O-sulfonylierten Produkte nicht aufweisen.

Wenn ausschließlich 1-Methyl-1 H-Imidazol in einer Menge von weniger als einem Äquivalent, bezogen auf das eingesetzte 3-Triazinyloxindol, eingesetzt wird, ist mit einer verminderten Ausbeute zu rechnen.

Jedoch wurde nun überraschend gefunden, dass eine selektive N-Sulfonylierung des Eduktes auch bei Verwendung einer Mischung möglich ist, welche aus einem 1-Alkyl-1 H-Imidazol und entweder einer tertiären cyclischen Aminbase wie Diazabicyclo[2.2.2]octan (DABCO), Methylmorpholin, 1-Ethylmorpholin, N,N-Dimethylpiperazin oder 1,2-Bis(dimethylamino)ethan oder einer substituierten Pyridinbasen wie 5-Ethyl-2-methylpyridin besteht und in der weniger als ein Äquivalent 1-Methyl-1 H-Imidazol, bezogen auf das eingesetzte 3-Triazinyloxindol, enthalten ist (Beispiel 1 Variante C).

Es liegt auch im Rahmen der Erfindung, dass neben den in 1-Position substituierten Imidazol-Basen ein Gemisch verschiedener Basentypen eingesetzt wird, wobei eine Basentype eine in 1-Position substituierte Imidazolbase ist. Bevorzugt ist ein Basen-Gemisch, das mindestens eine
- tertiäre Aminbase, oder
- substituierten Pyridinbase, und
zusätzlich mindestens eine der nachfolgenden, in 1-Position substituierten Imidazol-Basen
- 1-(C₁-C₆)Alky-1H-Imidazol,
- 1-Cycloalkyl-1H-Imidazol,
- 1-Benzyl-1 H-Imidazol,
- 1-Aryl-1 H-Imidazol, oder
- 1-Hetaryl-1H-Imidazol.
enthält.

Als teritäre Aminbasen werden bevorzugt Diazabicyclo[2.2.2]octan (DABCO), 1-Methylmorpholin, 1-Ethylmorpholin, N,N-Dimethylpiperazin oder 1,2-Bis(dimethylamino)ethan eingesetzt.

Als substituierte Pyridinbase in Mischungen mit einem oder mehreren 1-Alkyl-1 H-Imidazolen werden bevorzugt 5-Ethyl-2-methylpyridin oder 3-Methylpyridin oder ein Gemisch aus beiden eingesetzt.

Ein wichtiger Aspekt betrifft die Auswahl des Lösungsmittels, in dem die Umsetzung durchgeführt wird. Die Umsetzung kann in
- einem polaren, oder
- einem unpolaren Lösungsmittel, oder in
- einem Gemisch aus einem polaren oder unpolaren Lösungsmittel
durchgeführt werden.

Als unpolare Lösungsmittel können
- Aromaten, insbesondere Toluol, Xylol oder Chlorbenzol,
   verwendet werden.

Als polare organische Lösungsmittel können
- Haloalkane, insbesondere Dichlormethan oder Dichlorethan; oder
- Ketone, insbesondere Butanon, 2-Methylbutanon;
- Nitrile, insbesondere Acetonitril, Butyronitril, Isobutylnitril;
- Ether, insbesondere Dioxan, 2-Methyltetrahydrofuran, tert-Butylmethylether, Cyclopropylmethylether, Dimethoxyethan oder Tetrahydrofuran; oder
- Ester, insbesondere Ethylacetat, n-Butylacetat oder Isopropylacetat
verwendet werden.

Die genannten polaren Lösungsmittel können entweder allein oder in Mischungen mit anderen Lösungsmitteln, bevorzugt mit weiteren polaren organischen Lösungsmitteln, eingesetzt werden.

Besonders bevorzugt ist die Durchführung des Verfahrens mit Dichlormethan, 2-Methyltetrahydrofuran oder Ethylacetat als Lösungsmittel oder mit einem Gemisch, das mindestens eines der vorgenannten besonders bevorzugten Lösungsmittel enthält.

Das erfindungsgemäße Verfahren zur Herstellung von Verbindungen der Formel (3), basiert darauf, dass das 3-Triazinyloxindol (1 Äquivalent) in einem geeigneten Lösungsmittel mit der Sulfonylverbindung und einer Imidazol-Base, bzw. einem eine Imidazol-Base enthaltenden Gemisch, umgesetzt wird. Dabei wird die Sulfonylverbindung äquimolar oder im Überschuss (1.0 bis 6 Aquivalente, bevorzugt 1.2 bis 2 Äquivalente) eingesetzt. Ebenso wird die Base, entweder allein oder als Mischung aus mehreren Basen, äquimolar oder im Überschuss (1.0 bis 7 Äquivalente, bevorzugt 1.4 bis 2.5 Äquivalente) eingesetzt.

Die Zugabe der Reaktanden kann in einer Portion oder in mehreren Portionen über einen Zeitraum von bis zu 24 Stunden, bevorzugt bis zu 6 Stunden, insbesondere 0,05 bis 6 Stunden, erfolgen.

Die Reaktionstemperatur der Sulfonylierung liegt im Bereich von -100°C bis 50°C, bevorzugt im Bereich von -20°C bis +10°C.

Gegebenenfalls kann die Reaktion unter Druck durchgeführt werden.

Für gute Produktausbeuten ist es vorteilhaft wenn, zuerst das 3-Triazinyloxindol mit der Base (Gesamtmenge oder Teilmenge) in einem geeigneten Lösungsmittel vorgelegt und anschließend die Sulfonylverbindung und gegebenenfalls eine weitere Menge derselben oder einer anderen Base oder einer Mischung verschiedener Basen in einer oder mehreren Portionen zugeben wird.

Eine Zugabevariante besteht darin, dass 3-Triazinyloxindol und Sulfonylverbindung in einem geeigneten Lösungsmittel vorgelegt werden und danach die Base oder eine Mischung verschiedener Basen in einer oder mehreren Portionen zugegeben wird. Alternativ kann auch das 3-Triazinyloxindol in einer oder mehreren Portionen zur vorgelegten Sulfonylverbindung und der Base gegeben werden.

Es liegt im Rahmen der Erfindung, dass das 3-Triazinyloxindol als Salz in die Reaktionsmischung gegeben wird. In diesem Fall kann gegebenenfalls weniger Base eingesetzt werden.

Alle Reaktanden können entweder in reiner Form oder miteinander vorgemischt oder in einem Lösungsmittel oder einem Lösungsmittelgemisch gelöst oder suspendiert zur Reaktionsmischung gegeben werden. Es ist möglich, dass im Verlauf der Reaktion weiteres Lösungsmittel zugegeben wird, um eine bessere Durchmischung der Reaktanden zu ermöglichen.

Abhängig von den verwendeten Reaktionsbedingungen liegt die Nachrührzeit nach Zugabe aller Reaktanden im Bereich von bis zu 96 Stunden, bevorzugt 0,05 bis 24 Stunden.

Eine Nachrührzeit ist insbesondere dann vorteilhaft, wenn R³ = H ist, da gerade in diesem Fall bei der Sulfonylierungsreaktion zumindest teilweise auch das O-sulfonylierte Produkt gebildet werden kann, welches wiederum, wie oben erläutert, unter dem Einfluss einer Imidazol-Base zum gewünschten N-sulfonylierten Produkt der Formel (3) umgewandelt wird, bzw. weiterreagieren kann.

Aufarbeitung und Isolierung des gewünschten Produktes der Formel (3) kann auf verschiedene Weisen erfolgen und ist beispielsweise von der Wahl des Lösungsmittel abhängig oder ist davon abhängig, ob es sich bei dem Produkt um einen Feststoff oder um eine Flüssigkeit handelt.

Die Reaktionsmischung, welche ein festes Produkt der Formel (3) oder (3-1) enthält, wird filtriert. Das so erhaltene feste Produkt kann mit geeigneten Lösungsmitteln und /oder wässrigen Säuren gewaschen werden.

Es ist außerdem vorgesehen, dass zu der Reaktionsmischung, welche Produkt der Formel (3) enthält, ein anderes, höher siedendes Lösungsmittel gegeben wird, in welchem sich das Produkt schlechter löst, und das niedriger siedende Lösungsmittel ganz oder teilweise abdestilliert wird. Anschließend wird das als Feststoff vorliegende Produkt abfiltriert und kann mit geeigneten Lösungsmitteln und / oder wässrigen Säuren gewaschen werden.

Es liegt außerdem im Rahmen der Erfindung, dass das nach Filtration und optionalem Waschen erhaltene Produkt aus einem geeigneten Lösungsmittel oder einem Gemisch mehrerer Lösungsmittel ausgerührt wird, um eine höhere Reinheit zu erhalten.

Außerdem ist vorgesehen, dass die in der Reaktionsmischung enthaltenen Produkte der Formel (3), bzw. (3-1) oder deren Salze (3-1a) ohne Isolierung zu Folgeprodukten weiter umgesetzt werden.

Gegenstand der Erfindung sind auch N-sulfonylsubstituierte 3-Triazinyloxindole der Formel (3-1) oder deren Salze (3-1 a), unabhängig von der Art der Herstellung dieser Verbindungen.

Umfasst sind also N-sulfonylsubstituierte 3-Triazinyloxindole der Formel (3-1) 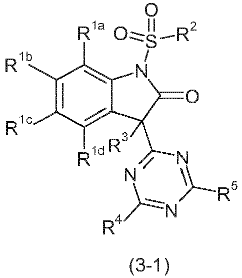 und deren Salze (3-1 a) 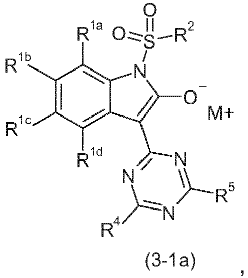 worin jeweils
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
Methyl, wobei das Methyl ganz oder teilweise mit Fluor substituiert ist, oder (C₃-C₇)-Cycloalkyl, wobei der Cycloalkylrest ganz oder teilweise mit Fluor substituiert ist,
steht,
R³ für
Wasserstoff, oder
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
steht,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
wobei in den Salzen der allgemeinen Formel (3-1a) M für Li, Na, K, Cs, Ba, Mg, Ca, Zn oder N(R^{c})₄ , worin R^{c} = H oder C₁-C₆ Alkyl ist, steht und wobei sich die Anzahl der Gegenionen M⁺ nach der jeweiligen Ladung richtet, so dass eine insgesamt neutrale Verbindung der allgemeinen Formel (3-1a) entsteht.

Von den Formeln (3-1) und (3-1 a) sind, soweit zutreffend, auch alle Stereoisomere, Rotamere, Tautomere und polymorphe Formen umfasst.

Besonders bevorzugt sind Verbindungen der Formel (3-1) oder Salze der Formel (3-1a), wobei
R^{1a} bis R^{1d} unabhängig voneinander ausgewählt sind aus der Gruppe bestehend aus
Wasserstoff, Fluor, Chlor sowie aus
(C₁-C₆)-Alkyl, wobei der Alkylrest verzweigt oder unverzweigt ist, oder
(C₁-C₆)-Alkoxy, wobei der Alkoxyrest verzweigt oder unverzweigt ist, und
R² für Difluormethyl oder Trifluormethyl steht,
R³ für Wasserstoff steht,
R⁴ und R⁵ unabhängig voneinander jeweils für
(C₁-C₄)-Alkyl, wobei der Alkylrest verzweigt oder unverzweigt ist,
(C₁-C₄)-Alkoxy, wobei der Alkoxyrest verzweigt oder unverzweigt ist, stehen,
wobei in den Salzen der allgemeinen Formel (3-1 a) M für Na und K steht.

Ganz besonders bevorzugt sind Verbindungen der Formel (3-1) oder deren Salze der Formel (3-1a), wobei
R^{1a} bis R^{1d} unabhängig voneinander ausgewählt sind aus der Gruppe bestehend aus Wasserstoff, Fluor, Chlor, Methoxy, und
R² für Difluormethyl steht,
R³ für Wasserstoff steht, und
R⁴ und R⁵ unabhängig voneinander jeweils für Methoxy stehen.

Am meisten bevorzugt sind Verbindungen der Formel (3-1) oder deren Salze der Formel (3-1a), wobei R^{1a} bis R^{1d} unabhängig voneinander ausgewählt sind aus der Gruppe bestehend aus Wasserstoff und Fluor.

Gegenstand der Erfindung ist auch die Verwendung der erfindungsgemäß hergestellten Verbindungen der Formeln (3) oder (3-1) oder deren Salze (3-1 a) als Wirkstoffe in der Landwirtschaft, sowie die Verwendung der genannten Verbindungen zur Herstellung von Wirkstoffen für die Landwirtschaft oder zur Herstellung von Zwischenprodukten zur Herstellung von Feinchemikalien und Wirkstoffen für die Landwirtschaft.

Besonders bevorzugt ist die Verwendung als Fungizid oder Herbizid, bzw. die Herstellung von Fungiziden und Herbiziden, bzw. die Verwendung der genannten Verbindungen als Zwischenprodukte zur Herstellung von Fungiziden und Herbiziden.

Die Verwendung der genannten Verbindungen als Herbizid, bzw. die Herstellung von Herbiziden ist ganz besonders bevorzugt.

N-sulfonylsubstituierte 3-Triazinyloxinodole der allgemeinen Formeln (3-1) und (3-1a) sowie auch die übrigen nach dem erfindungsgemäßen Verfahren hergestellten N-sulfonylsubstituierten 3-Heteroaryloxindole der allgemeinen Formel (3) eignen sich als Zwischenprodukte zur Herstellung von Feinchemikalien und Wirkstoffen für die Landwirtschaft.

Hergestellt werden können daraus N-Alkyl-N-[2-(1,3,5-triazin-2-ylcarbonyl)phenyl]alkansulfonamide der allgemeinen Formel (4-1) - siehe nachfolgendes Schema 4 -, deren herbizide Aktivität (siehe WO 2007/031208 A2) und fungizide Aktivität (siehe WO 2006/008159 A1) in den beiden genannten Offenlegungsschriften gezeigt wird.

Verbindungen der Formel (3) sind N-sulfonylsubstituierte 3-Triazinyloxindole.

Im nachfolgenden Schema 4 sind N-sulfonylsubstituierte 3-Triazinyloxindole mit der Formel (3-1) bezeichnet.

Schema 4 zeigt ein neues mehrstufiges Syntheseverfahren, gemäß dem, ausgehend von einem 3-(Alkylsulfanyl)-1,3-dihydro-2H-indol-2-on der Formel (7-1) in einer insgesamt fünfstufigen Reaktion ein N-Alkyl-N-[2-(1,3,5-triazin-2-ylcarbonyl)phenyl]alkansulfonamid der Formel (4-1), dessen herbizide (siehe WO 2007/031208 A2) und fungizide (siehe WO 2006/008159 A1) Aktivität bereits seit längerem bekannt ist, hergestellt werden kann.

### Schema 4: Mehrstufiges Verfahren zur Herstellung von für den Pflanzenschutz geeigneten, insbesondere herbiziden, N-Alkyl-N-[2-(1,3,5-triazin-2-ylcarbonyl)phenyl]-alkansulfonamiden (4-1)

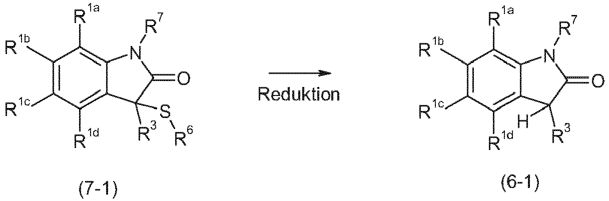

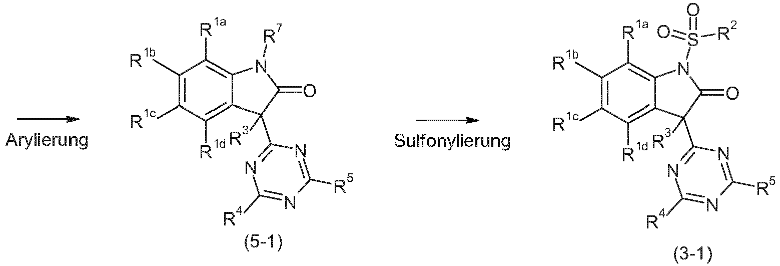

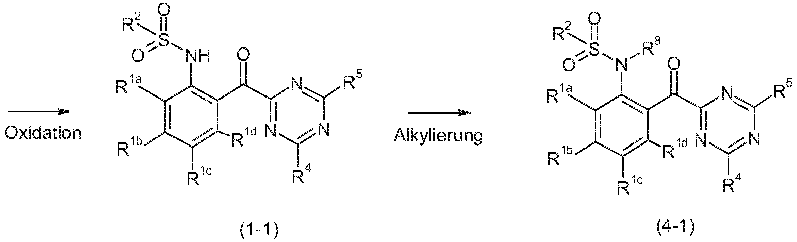

Das mehrstufige Verfahren gemäß Schema 4 zur Herstellung von N-Alkyl-N-[2-(1,3,5-triazin-2-ylcarbonyl)phenyl]alkansulfonamiden (4-1) besteht aus folgenden Teilschritten:
- Reduktion von substituierten oder unsubstituierten 3-(Alkylsulfanyl)-1,3-dihydro-2H-indol-2-onen (7-1) zu substituierten oder unsubstituierten 1,3-Dihydro-2H-indol-2-onen (6-1). Dieses Verfahren ist im technischen Maßstab möglich und ist in der Patentanmeldung mit der Anmeldenummer EP 10162381.7 beschrieben.
- Arylierung von substituierten oder unsubstituierten 1,3-Dihydro-2H-indol-2-onen (6-1) zu triazinylsubstituierten Oxindolen (5-1). Dieses Verfahren ist im technischen Maßstab möglich und ist in der Patentanmeldung mit der Anmeldenummer EP 10196205.8 beschrieben.
- Sulfonylierung von triazinylsubstituierten Oxindolen (5-1) zu N-sulfonylsubstituierten 3-Triazinyloxindolen (3-1) gemäß dem vorliegenden erfindungsgemäßen Sulfonylierungsverfahren.
- Oxidative Ringöffnung von N-sulfonylsubstituierten 3-Triazinyloxindolen (3-1) zu 2-(Triazinylcarbonyl)sulfonaniliden (1-1). Dieses Verfahren ist im technischen Maßstab möglich und ist in der Patentanmeldung DE 102011086382.6 beschrieben.
- Alkylierung von 2-(Triazinylcarbonyl)sulfonaniliden (1-1) zu N-Alkyl-N-[2-(1,3,5-triazin-2-ylcarbonyl)phenyl]alkansulfonamiden (4-1). Dieses Verfahren ist in der Patentanmeldung mit der Anmeldenummer WO 2006/008159 A1 beschrieben.

Das in Schema 4 dargestellte mehrstufige Verfahren zeichnet sich gegenüber den vorbekannten Verfahren zur Herstellung von N-Alkyl-N-[2-(1,3,5-triazin-2-ylcarbonyl)phenyl]-alkansulfonamiden (4-1) sowie 2-(Triazinylcarbonyl)sulfonaniliden (1-1) dadurch aus, dass Oxindolverbindungen als Edukte bzw. als Intermediate eingesetzt werden. Dies bringt den Vorteil, dass das Verfahren im Vergleich zu den vorbekannten Verfahren im technischen Maßstab durchgeführt werden kann und zugleich hohe Ausbeuten erhalten werden können.

Die Ausführbarkeit des in Schema 4 zusammengefassten Verfahrens ist im Detail nachfolgend offenbart. Die Reduktion, die in Schema 4 den ersten Reaktionsschritt des insgesamt fünfstufigen Verfahrens betrifft, wurde nachfolgend als eigenständige Vorstufe B) behandelt. Das nachfolgend im Detail beschriebene Verfahren A) umfasst somit nur die Schritte der Arylierung, der Sulfonylierung, der Oxidation und der Alkylierung.

### A) Verfahren zur Herstellung von N-Alkyl-N-[2-(1,3,5-triazin-2-ylcarbonyl)phenyl]alkansulfonamiden der Formel (4-1)

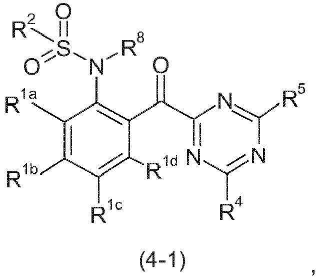

worin
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-Cₛ)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, und Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
R⁴ und R⁵ unabhängig voneinander jeweils für
   Wasserstoff,
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   stehen, und
R⁸ für
   (C₁-C₆)-Alkyl, wobei der Alkylrest unsubstituiert ist oder ganz oder teilweise mit Fluor substituiert ist,
   (C₁-C₆)-cycloalkyl, (C₁-C₆)-Alkenyl oder (C₁-C₆)-Alkoxyalkyl, wobei jeder der genannten Reste unsubstituiert ist oder ganz oder teilweise mit Fluor substituiert ist,
   steht, wobei
ein 1,3-Dihydro-2H-indol-2-on der Formel (6-1) 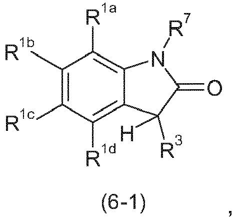 worin
R^{1a} bis R^{1d} wie für Formel (4-1) definiert sind,
R³ für Wasserstoff steht, und
R⁷ für Wasserstoff steht, in einem
ersten Schritt durch
- Arylierung zu einem triazinylsubstituierten Oxindol der Formel (5-1) 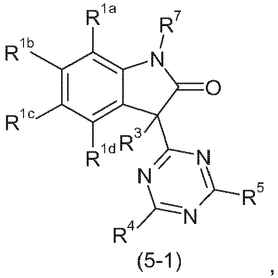 worin
   R^{1a} bis R^{1d} und R⁴ und R⁵ wie für die Formel (4-1) und R³ und R⁷ wie für die Formel (6-1) definiert sind,
   umgesetzt wird, und die Arylierungsprodukte der Formel (5-1) in einem zweiten Schritt durch
- Sulfonylierung zu N-sulfonylsubstituierten 3-Triazinyloxindolen der Formel (3-1) 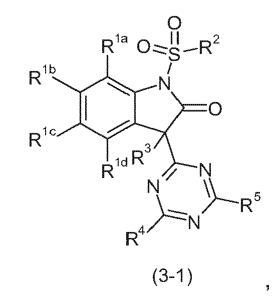 worin
   R^{1a} bis R^{1d}, R² sowie R⁴ und R⁵ wie in Formel (4-1) und R³ wie für die Formel (6-1)definiert sind,
   umgesetzt werden, und die Sulfonylierungsprodukte der Formel (3-1) in einem
dritten Schritt durch
- oxidative Ringöffnung zu einem 2-(Triazinylcarbonyl)sulfonanilid der Formel (1-1) 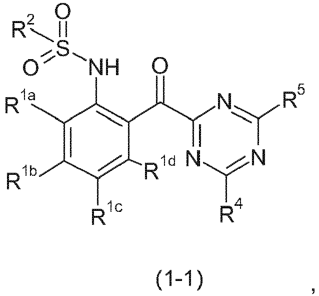 worin
   R^{1a} bis R^{1d}, R² sowie R⁴ und R⁵ wie für Formel (4-1) definiert sind,
   umgesetzt werden, und die Oxidationsprodukte der Formel (1-1) in einem vierten Schritt durch
- Alkylierung zu einem N-Alkyl-N-[2-(1,3,5-triazin-2-ylcarbonyl)phenyl] alkansulfonamid der Formel (4-1) 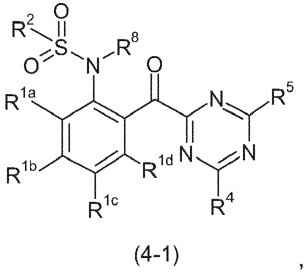 worin
   R^{1a} bis R^{1d}, R², R⁴, R⁵ und R⁸ wie oben für Formel (4-1) definiert sind,
   umgesetzt werden, wobei als Alkylierungsreagenz
   - X- R⁸, wobei X für Chlor, Brom oder Iod steht und R⁸ wie oben für Formel (4-1) definiert ist, oder
   - (R⁸)₂SO₄ , worin R⁸ wie oben für Formel (4-1) definiert ist,
eingesetzt wird.

Die Sulfonylierung erfolgt nach der Lehre der vorliegenden Erfindung, d.h. in Gegenwart
- einer in 1-Position substituierten Imidazol-Base, oder
- eines Basen-Gemischs, das mindestens eine in 1-Position substituierte Imidazol-Base enthält.

Besonders bevorzugte Imidazol-Basen sind 1-Methyl-1 H-Imidazol, 1-Butyl-1 H-Imidazol oder 1-Benzyl-1 H-Imidazol, die einzeln oder im Gemisch eingesetzt werden können, wobei die Verwendung von 1-Methyl-1 H-Imidazol ganz besonders bevorzugt ist.

B) Verfahren zur Herstellung von N-Alkyl-N-[2-(1,3,5-triazin-2-ylcarbonyl)phenyl] alkansulfonamiden der Formel (4-1) wobei die als Edukt eingesetzten Verbindungen der Formel (6-1) in einem vorhergehenden Verfahrensschritt hergestellt werden, bei welchem ausgehend von einem 3-(Alkylsulfanyl)-1,3-dihydro-2H-indol-2-on der Formel (7-1), 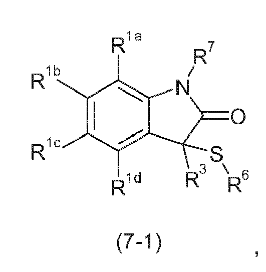 worin
R^{1a} bis R^{1d} wie für Formel (4-1) definiert sind,
R³ für Wasserstoff steht,
R⁷ für Wasserstoff steht, und
R⁶ ein unsubstituiertes oder substituiertes (C₁-C₁₄)-Alkyl, (C₃-C₇)-Cycloalkyl, Benzyl oder ein CH₂-C(O)O-(C₁-C₆)-Alkyl ist,
durch
- Reduktion zu einem 1,3-Dihydro-2H-indol-2-on (6-1) 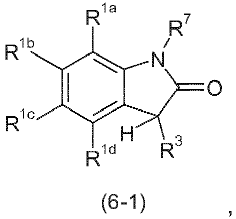 worin
   R^{1a} bis R^{1d}, R³ und R⁷ wie für Formel (7-1) definiert sind,
umgewandelt wird.

Bei der Durchführung der Reduktion wird
a) eine Verbindung der Formel (7-1) in einem polaren Lösungsmittel gelöst oder suspendiert,
b) der Lösung oder der Suspension ein schwefelhaltiges Salz zugesetzt, und
c) das Reaktionsgemisch bei einer Temperatur, die maximal der Siedetemparatur des polaren Lösungsmittels entspricht, unter Rückfluss erwärmt.

Als schwefelhaltige Salze besonders bevorzugt sind Natriumsalze, ausgewählt aus der Gruppe bestehend aus Natriumbisulfit, Natriumsulfit, Natriumthionit, Natriumdithionit und Natriumthiosulfat.

Die herbizide Wirkung (siehe WO 2007/031208 A2) und fungizide Wirkung (siehe WO 2006/008159 A1) von N-Alkyl-N-[2-(1,3,5-triazin-2-ylcarbonyl)phenyl]alkansulfonamiden der Formel (4-1) ist, wie bereits erwähnt, seit längerem bekannt.

Somit wird durch Schema 44 und die Ausführbarkeit der Verfahren A) und B) belegt, dass heteroarylsubstituierte Oxindole der Formel (3), bzw. triazinylsubstituierte Oxindole der Formel (3-1) oder deren Salze (3-1a)als Intermediate zur Herstellung von Pflanzenschutzmitteln, insbesondere von Herbiziden und Fungiziden, geeignet sind.

Die Verwendung von Verbindungen der Formeln (3) oder (3-1) oder deren Salze (3-1a) als Zwischenprodukte zur Herstellung von N-[2-(1,3,5-Triazin-2-ylcarbonyl)phenyl]alkylsulfonamiden ist am meisten bevorzugt. BEISPIELE

Die nachfolgenden Beispiele erläutern die Erfindung näher, jedoch ohne deren Gegenstand auf diese Beispiele einzuschränken.

In den nachfolgenden Beispielen beziehen sich Mengenangaben auf das Gewicht, sofern nichts anderes speziell definiert ist (in der Beschreibung wurde hierfür analog Gew.% = Gewichtsprozent verwendet). Für Maßeinheiten, physikalische Größen und ähnliches werden übliche Abkürzungen verwendet, beispielsweise h = Stunde(n), mpt. = Schmelzpunkt (Smp.), I = Liter, ml = Milliliter, g = Gramm, min = Minute(n), *in vacuo* = "im Vakuum" = unter reduziertem Druck, d. Th. = Prozent Ausbeute nach der Theorie, RT = Raumtemperatur, eq. = Äquivalente.

Die Kupplungsmuster in den NMR-Spektren werden so beschrieben, wie sie erscheinen.

Anteile in der HPLC-Analytik werden, wenn nichts anderes angegeben ist, in relativen Flächenprozenten wiedergegeben.

Prozentangaben in der LCMS-Analytik beziehen sich auf den relativen Anteil der jeweiligen Komponente im Chromatogramm.

### Beispiel 1:

### Herstellung von 1-[(Difluormethyl)sulfonyl]-3-(4,6-dimethoxy-1,3,5-triazin-2-yl)-7-fluor-1,3-dihydro-2H-indol-2-on

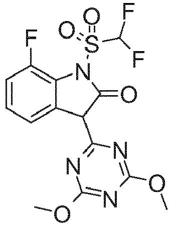

### Variante A:

3-(4,6-Dimethoxy-1,3,5-triazin-2-yl)-7-fluor-1,3-dihydro-2H-indol-2-on (95,7g) und 1-Methyl-1 H-Imidazol (53, 1g) werden in 1120 ml Ethylacetat vorgelegt und unter Stickstoff auf 0°C gekühlt. Unter kräftigen Rühren wird Difluormethansulfonylchlorid (73,7g) bei 0°C bis 5°C innerhalb 30 Minuten zugetropft und 3,5 Stunden nachgerührt. Man gibt weiteres Difluormethansulfonylchlorid (4,9g) zur Reaktionsmischung und rührt 1,5 Stunden. Die Reaktionsmischung wird mit 500 ml Wasser versetzt und kräftig durchgemischt. Das organische Lösungsmittel wird im Vakuum weitgehend abdestilliert und der Rückstand filtriert. Das feste Produkt wird zweimal mit je 250 ml Wasser gewaschen, aus 400 ml Ethylacetat/ Heptan (1:1) ausgerührt, abfiltriert und mit 100 ml Ethylacetat/ Heptan (1:1) gewaschen. Man erhält die Titelverbindung als Feststoff in einer Reinheit von 96% gegen Standard (123,1g, 91% d. Th). Die UV-Absorption (Maximum bei 360 nm) belegt die N-Sulfonylierung des Produktes.
LC-MS: M+H = 405 (95%).
1 H-NMR (400 MHz, CDCl₃): δ (ppm) = 12.6 (s, breit, 1 H), 7.83 (d, 1 H), 7.20 (dt, 1 H), 6.93 (dd, 1H), 6.70 (t, 1H), 4.21 (s, 3H), 4.16 (s, 3H).

### Variante B:

3-(4,6-Dimethoxy-1,3,5-triazin-2-yl)-7-fluor-1,3-dihydro-2H-indol-2-on (100g) und 1-Methyl-1 H-Imidazol (44,8g) werden in 670 ml Dichlormethan vorgelegt und unter Stickstoff auf -10°C gekühlt. Unter kräftigen Rühren wird Difluormethansulfonylchlorid (72,6g) bei -12°C bis -3°C innerhalb 35 Minuten zugetropft und 4,5 Stunden bei -10°C bis -5°C nachgerührt. Eine HPLC-Kontrolle nach einer Stunde bzw. 3 Stunden zeigt das Vorhandensein des als Zwischenprodukt entstandenen O-sulfonylierten Eduktes an, welches zum Reaktionsende nahezu vollständig in das gewünschte Produkt umgewandelt wurde. Die Reaktionsmischung wird mit 500 ml Wasser versetzt und kräftig durchgemischt. Das organische Lösungsmittel wird im Vakuum weitgehend abdestilliert und der Rückstand filtriert. Das feste Produkt wird zweimal mit je 100 ml fünfprozentiger Salzsäure und zweimal mit je 100 ml Wasser gewaschen und im Vakuumtrockenschrank getrocknet. Man erhält die Titelverbindung als Feststoff in einer Reinheit von 93% gegen Standard (136g, 93% d. Th). Die NMR-Signale des Produktes stimmen mit den Signalen des nach Variante A erhaltenen Produktes überein.

### Variante C:

3-(4,6-Dimethoxy-1,3,5-triazin-2-yl)-7-fluor-1,3-dihydro-2H-indol-2-on (10g; 33 mmol) wird in 40 ml Dichlormethan vorgelegt und auf -20°C gekühlt. 1-Methyl-1H-imidazol (0,84g; 10 mmol) sowie 4-Ethylmorpholin (5,5g, 47 mmol) werden hinzugegeben und die Reaktionsmischung kurz gerührt. Unter kräftigen Rühren wird Difluormethansulfonylchlorid (6,2g) bei -20°C bis -10°C zugetropft und 3 Stunden bei -20°C bis -10°C nachgerührt. Es wird mit 50 ml Dichlormethan versetzt und eine Stunde nachgerührt. Difluormethansulfonylchlorid (0,5g) wird zugegeben und eine Stunde nachgerührt. Die Reaktionsmischung wird auf Raumtemperatur erwärmt, in einen Ausrührkolben überführt und mit 100 ml Wasser versetzt. Das organische Lösungsmittel wird im Vakuum weitgehend abdestilliert und der Rückstand filtriert. Das feste Produkt wird mit je 50 ml fünfprozentiger Salzsäure, Wasser und 2-Propanol gewaschen und im Vakuumtrockenschrank getrocknet. Man erhält die Titelverbindung als Feststoff in einer HPLC-Reinheit von 98,8% (13,3g, 97% d. Th). Die NMR-Signale des Produktes stimmen mit den Signalen des nach Variante A erhaltenen Produktes überein.

### Variante D (= Vergleichsbeispiel 1 D):

3-(4,6-Dimethoxy-1,3,5-triazin-2-yl)-7-fluor-1,3-dihydro-2H-indol-2-on (3,0g) wird in 60 ml Tetrahydrofuran vorgelegt, auf 0°C gekühlt und Natriumhydrid (0,52g, 60% in Mineralöl) zugegeben. Die Reaktionsmischung wird eine Stunde gerührt, Difluormethansulfonylchlorid (1,7g) bei 0°C zugetropft und 12 Stunden bei 0°C nachgerührt. Durch HPLC-Analytik werden Edukt (72%), das gewünschte Produkt (8%) und weitere Komponenten detektiert.

### Variante E (= Vergleichsbeispiel 1 E):

3-(4,6-Dimethoxy-1,3,5-triazin-2-yl)-7-fluor-1,3-dihydro-2H-indol-2-on (3,0g) wird in 60 ml Tetrahydrofuran vorgelegt, auf-30°C gekühlt und Kalium-tert.-butylat (1,26g) zugegeben. Die Reaktionsmischung wird innerhalb einer Stunde auf 0°C erwärmt, anschließend auf -60°C gekühlt, Difluormethansulfonylchlorid (1,7g) zugetropft, langsam auf Raumtemperatur erwärmt und 12 Stunden nachgerührt. Durch HPLC-Analytik werden Edukt (61 %), das gewünschte Produkt (6%) und weitere Komponenten detektiert.

### Variante F (= Vergleichsbeispiel 1 F):

3-(4,6-Dimethoxy-1,3,5-triazin-2-yl)-7-fluor-1,3-dihydro-2H-indol-2-on (3,0g) wird in 30 ml Dichlormethan vorgelegt, auf -15°C gekühlt und Triethylamin (1,86g) zugegeben. Die Reaktionsmischung wird kurz gerührt, Difluormethansulfonylchlorid (2,2g) zugetropft und 3 Stunden bei -15 bis -10°C nachgerührt. Der Ansatz steht 15 Stunden bei Raumtemperatur. Durch HPLC-Analytik werden Edukt (53%), das gewünschte Produkt (ca. 3%) und weitere Komponenten detektiert.

### Variante G:

3-(4,6-Dimethoxy-1,3,5-triazin-2-yl)-7-fluor-1,3-dihydro-2H-indol-2-on (3g; 10 mmol) wird in 50 ml Dichlormethan vorgelegt. 1-Methyl-1H-imidazol (0,17g; 2,0 mmol) sowie 5-Ethyl-2-methylpyridin (2,0g, 16 mmol) werden hinzugegeben, die Reaktionsmischung auf -10°C gekühlt und kurz gerührt. Unter kräftigen Rühren wird Difluormethansulfonylchlorid (2,5g, 16 mmol) bei -20°C bis -5°C zugetropft und 7 Stunden bei -20°C bis -5°C nachgerührt. Die Reaktionsmischung wird auf Raumtemperatur erwärmt, in einen Ausrührkolben überführt und mit 30 ml Wasser versetzt. Das organische Lösungsmittel wird im Vakuum weitgehend abdestilliert und der Rückstand filtriert. Das feste Produkt wird mit je 30 ml fünfprozentiger Salzsäure, Wasser und 2-Propanol gewaschen und im Vakuumtrockenschrank getrocknet. Man erhält die Titelverbindung als Feststoff in einer HPLC-Reinheit von 94% (3,7g, 84% d. Th).

### Beispiel 2:

### Herstellung von 1-[(Difluormethyl)sulfonyl]-7-fluor-3-(4-methoxy-6-methyl-1,3,5-triazin-2-yl)-1,3-dihydro-2H-indol-2-on

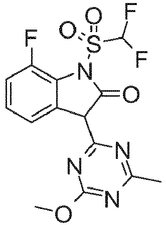

7-Fluor-3-(4-methoxy-6-methyl-1,3,5-triazin-2-yl)-1,3-dihydro-2H-indol-2-on (1,20g) und 1-Methyl-1H-Imidazol (0,97g) werden in 10 ml Dichlormethan vorgelegt und unter Stickstoff auf 0°C gekühlt. Unter Rühren wird Difluormethansulfonylchlorid (1,21g) in 2 Portionen zugegeben und der Ansatz auf Raumtemperatur erwärmt. Nach 5 Stunden werden 1-Methyl-1H-Imidazol (0,32g) und Difluormethansulfonylchlorid (0,61g) zugegeben und 16 Stunden gerührt. Nach Zugabe von Wasser und etwas Salzsäure wird der Feststoff abfiltriert, mit Dichlormethan und Wasser gewaschen und getrocknet. Man erhält die Titelverbindung als Feststoff in einer HPLC-Reinheit von 91% (1,54g, 92% d. Th). Die UV-Absorption (Maximum bei 360 nm) belegt die N-Sulfonylierung des Produktes.
LC-MS: M+H = 389 (71%).
1 H-NMR (400 MHz, DMSO-D₆): δ (ppm) = 7.94 (d, 1 H), 7.53 (t, 1 H), 7.29 (dt, 1 H), 7.03 (dd, 1 H), 4.12 (s, 3H), 2.58 (s, 3H).

### Beispiel 3:

### Herstellung von 1-[(Difluormethyl)sulfonyl]-3-(4,6-dimethoxy-1,3,5-triazin-2-yl)-1,3-dihydro-2H-indol-2-on

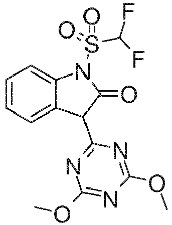

3-(4,6-Dimethoxy-1,3,5-triazin-2-yl)-1,3-dihydro-2H-indol-2-on (10g) und 1-Methyl-1 H-Imidazol (5,4g) werden in 100 ml Dichlormethan vorgelegt und unter Stickstoff auf -5°C gekühlt. Unter Rühren wird Difluormethansulfonylchlorid (7,03g) bei -5°C bis 0°C innerhalb 20 Minuten zugetropft und 2,5 Stunden bei dieser Temperatur nachgerührt. Die Reaktionsmischung wird mit 50 ml Wasser versetzt und durchgemischt. Das organische Lösungsmittel wird im Vakuum weitgehend abdestilliert und der Rückstand filtriert. Das feste Produkt wird mit Wasser und Acetonitril gewaschen und im Vakuumtrockenschrank getrocknet. Man erhält die Titelverbindung als Feststoff in einer HPLC-Reinheit von 93% (12,5g, 92% d. Th). Die UV-Absorption (Maximum bei 360 nm) belegt die N-Sulfonylierung des Produktes.
LC-MS: M+H = 387 (98%).
1H-NMR (400 MHz, CDCl₃): δ (ppm) = 12.55 (s, breit, 1H), 7.98 (d, 1H), 7.76 (d, 1H), 7.23 (t, 1H), 7.16 (t, 1H), 6.68 (t, 1H), 4.22 (s, 3H), 4.15 (s, 3H).

### Beispiel 4:

### Herstellung von 1-[(Difluormethyl)sulfonyl]-3-(4,6-dimethoxy-1,3,5-triazin-2-yl)-5-fluor-1,3-dihydro-2H-indol-2-on

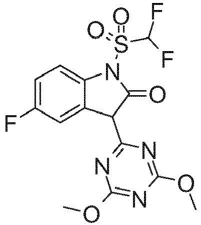

3-(4,6-Dimethoxy-1,3,5-triazin-2-yl)-5-fluor-1,3-dihydro-2H-indol-2-on (50g) und 1-Methyl-1 H-Imidazol (30,5g) werden in 430 ml Dichlormethan vorgelegt und unter Stickstoff auf -10°C gekühlt. Unter Rühren wird eine Lösung von Difluormethansulfonylchlorid (41,8g) in 70 ml Dichlormethan bei -15°C bis -5°C innerhalb 30 Minuten zugetropft und 4 Stunden bei -15°C bis -0°C nachgerührt. Man gibt 1-Methyl-1 H-Imidazol (2,7g) und Difluormethansulfonylchlorid (4,9g) zur Reaktionsmischung und rührt 2 Stunden bei 0°C. Unter Eiskühlung wird 300 ml Wasser zur Reaktionsmischung gegeben, 30 Minuten nachgerührt und das organische Lösungsmittel wird im Vakuum weitgehend abdestilliert. Der Rückstand wird filtriert, das feste Produkt zweimal mit 150 ml Wasser und zweimal mit 40 ml Acetonitril gewaschen und getrocknet. Man erhält die Titelverbindung als Feststoff in einer HPLC-Reinheit von 96% (63,9g, 95% d. Th). Die UV-Absorption (Maximum bei 360 nm) belegt die N-Sulfonylierung des Produktes.
LC-MS: M+H = 405 (97%).
1 H-NMR (400 MHz, CDCl₃): δ (ppm) = 12.5 (s, breit, 1 H), 7.63-7.72 (m, 2H), 6.85 (dt, 1 H), 6.67 (t, 1 H), 4.22 (s, 3H), 4.17 (s, 3H).

### Beispiel 5:

### Herstellung von 3-(4,6-Diethoxy-1,3,5-triazin-2-yl)-1-[(difluormethyl)sulfonyl]-7-fluor-1,3-dihydro-2H-indol-2-on

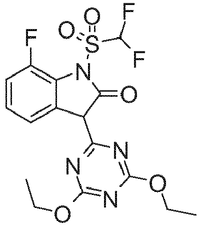

3-(4,6-Diethoxy-1,3,5-triazin-2-yl)-7-fluor-1,3-dihydro-2H-indol-2-on (1,0g) und 1-Methyl-1 H-Imidazol (0,51g) werden in 10 ml Dichlormethan vorgelegt und unter Stickstoff auf 0°C gekühlt. Unter Rühren wird Difluormethansulfonylchlorid (0.46g) zugetropft und 2 Stunden bei 0°C bis 6°C nachgerührt. Nach Zugabe von 1-Methyl-1 H-Imidazol (0,25g) und Difluormethansulfonylchlorid (0,23g) wird 2 Stunden bei 0°C bis 10°C gerührt. Nach Zugabe von 5 ml Wasser wird mit zehnprozentiger Salzsäure auf pH 2 gestellt, das organische Lösungsmittel im Vakuum weitgehend abdestilliert, der Feststoff abfiltriert, mit Wasser und Heptan gewaschen und getrocknet. Man erhält die Titelverbindung als Feststoff in einer HPLC-Reinheit von 93% (1,19g, 83% d. Th). Die UV-Absorption (Maximum bei 360 nm) belegt die N-Sulfonylierung des Produktes.
LC-MS: M+H = 433 (91%).
1 H-NMR (400 MHz, DMSO-D₆): δ (ppm) = 7.86 (dd, 1 H), 7.52 (t, 1 H), 7.22-7.31 (m, 1H), 7.00 (dd, 1H), 4.58 (q, 4H), 1.41 (t, 6H).

### Beispiel 6:

### Herstellung von 1-[(Difluormethyl)sulfonyl]-3-(4,6-dimethoxy-1,3,5-triazin-2-yl)-5,7-difluor-1,3-dihydro-2H-indol-2-on

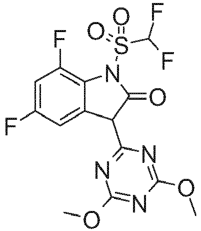

3-(4,6-Dimethoxy-1,3,5-triazin-2-yl)-5,7-difluor-1,3-dihydro-2H-indol-2-on (1,0g), 1-Methyl-1 H-Imidazol (0,74g) und Difluormethansulfonylchlorid (1,09g) werden in 8 ml Dichlormethan analog zu Beispiel 5 umgesetzt. Zur Aufarbeitung wird Wasser zugegeben und gerührt, der Feststoff abfiltriert, mit verdünnter Salzsäure und Wasser gewaschen und getrocknet. Man erhält die Titelverbindung als Feststoff in einer HPLC-Reinheit von 97% (1,08g, 82% d. Th). Die UV-Absorption (Maximum bei 360 nm) belegt die N-Sulfonylierung des Produktes.
LC-MS: M+H = 423 (96%).
1 H-NMR (400 MHz, DMSO-D₆): δ (ppm) = 7.64 (dd, 1 H), 7.51 (t, 1 H), 7.00 (dt, 1 H), 4.12 (s, 6H).

### Beispiel 7:

### Herstellung von 1-[(Difluormethyl)sulfonyl]-3-(4,6-dimethoxy-1,3,5-triazin-2-yl)-5-methoxy-1,3-dihydro-2H-indol-2-on

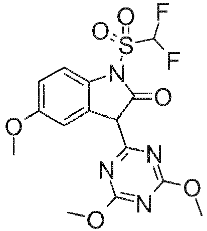

3-(4,6-Dimethoxy-1,3,5-triazin-2-yl)-5-methoxy-1,3-dihydro-2H-indol-2-on (1,0g) und 1-Methyl-1H-Imidazol (0,51g) werden in 8 ml Dichlormethan vorgelegt und unter Stickstoff auf -10°C gekühlt. Unter Rühren wird Difluormethansulfonylchlorid (0.66g) in zwei Portionen zugetropft und der Ansatz 2,5 Stunden bei -5 bis -10°C gerührt. Zur Aufarbeitung wird Wasser zugegeben und gerührt, der Feststoff abfiltriert, mit verdünnter Salzsäure und Wasser gewaschen und getrocknet. Man erhält die Titelverbindung als Feststoff in einer HPLC-Reinheit von 99% (0,91g, 70% d. Th). Die UV-Absorption (Maximum bei 360 nm) belegt die N-Sulfonylierung des Produktes.
LC-MS: M+H = 417 (100%).
1 H-NMR (400 MHz, DMSO-D₆): δ (ppm) = 7.61 (d, 1 H), 7.46 (d, 1H), 7.44 (t, 1H), 6.71 (dd, 1 H), 4.11 (s, 6H).3.77 (s, 3H).

### Beispiel 8:

### Herstellung von 1-[(Difluormethyl)sulfonyl]-3-(4,6-dimethoxy-1,3,5-triazin-2-yl)-7-methoxy-1,3-dihydro-2H-indol-2-on

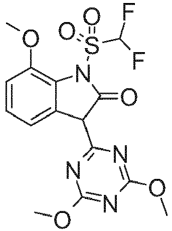

3-(4,6-Dimethoxy-1,3,5-triazin-2-yl)-7-methoxy-1,3-dihydro-2H-indol-2-on (1,0g) und 1-Methyl-1H-Imidazol (0,47g) werden in 8 ml Dichlormethan vorgelegt und unter Stickstoff auf -15°C gekühlt. Unter Rühren wird Difluormethansulfonylchlorid (0,62g) in zwei Portionen zugetropft und der Ansatz in 2.5 Stunden auf 10°C erwärmt. Es wird auf 0°C gekühlt, 1-Methyl-1H-Imidazol (0,24g) und Difluormethansulfonylchlorid (0,35g) zugegeben, innerhalb 3 Stunden auf Raumtemperatur erwärmt, 4 Stunden nachgerührt und 48 Stunden stehen gelassen. Zur Aufarbeitung wird Wasser zugegeben und gerührt, der Feststoff abfiltriert, mit verdünnter Salzsäure und Wasser gewaschen und getrocknet. Man erhält die Titelverbindung als Feststoff in einer HPLC-Reinheit von 91% (0,52g, 39% d. Th). Die UV-Absorption (Maximum bei 360 nm) belegt die N-Sulfonylierung des Produktes.
LC-MS: M+H = 417 (87%).
1 H-NMR (400 MHz, DMSO-D₆): δ (ppm) = 7.68 (d, 1 H), 7.46 (t, 1 H), 7.23 (t, 1 H), 6.90 (d, 1 H), 4.11 (s, 6H), 3.88 (s, 3H).

### Beispiel 9:

### Herstellung von 3-(4,6-Dimethoxy-1,3,5-triazin-2-yl)-7-fluor-1-[(trifluormethyl)sulfonyl]-1,3-dihydro-2H-indol-2-on

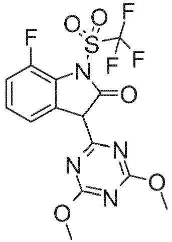

3-(4,6-Dimethoxy-1,3,5-triazin-2-yl)-7-fluor-1,3-dihydro-2H-indol-2-on (1,0g) und 1-Methyl-1 H-Imidazol (1,09g) werden in 10 ml Dichlormethan vorgelegt und unter Stickstoff auf 0°C gekühlt. Unter Rühren wird Trifluormethansulfonsäureanhydrid (1,89g) in 4 Portionen bei 0-10°C Innentemperatur zugegeben. Man rührt eine Stunde bei max. 15°C. Der Ansatz wird wieder im Eisbad gekühlt, Wasser zugegeben, mit Salzsäure auf pH 2 gestellt und die Phasen getrennt. Die organische Phase wird mit Wasser gewaschen, getrocknet und eingeengt. Man erhält die Titelverbindung als Feststoff in einer HPLC-Reinheit von 68% (1,38g, 67% d. Th). Die UV-Absorption (Maximum bei 360 nm) belegt die N-Sulfonylierung des Produktes.
LC-MS: M+H = 423 (35%)
1 H-NMR (400 MHz, DMSO-D₆): δ (ppm) = 7.96 (d, 1 H), 7.32 (dt, 1 H), 7.03 (dd, 1 H), 4.12 (s, 6H).

### Beispiel 10:

### Herstellung von 3-(4,6-Dimethoxy-1,3,5-triazin-2-yl)-7-fluor-1 H-indol-2-yltrifluormethansulfonat

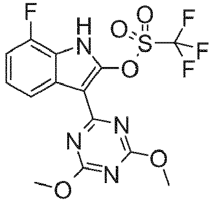

3-(4,6-Dimethoxy-1,3,5-triazin-2-yl)-7-fluor-1,3-dihydro-2H-indol-2-on (1,0g) und 1,4-Diazabicyclo[2.2.2]octan (0,82g) werden in 10 ml Dichlormethan vorgelegt und unter Stickstoff auf -10°C gekühlt. Unter Rühren wird Trifluormethansulfonsäureanhydrid (1,42g) zugetropft und der Ansatz in 4,5 Stunden auf Raumtemperatur erwärmt. Der Ansatz steht 15 Stunden. Man gibt 1,4-Diazabicyclo[2.2.2]octan (0,48g) und Trifluormethan-sulfonsäureanhydrid (0,94g) hinzu, rührt 5 Stunden und stellt mit verdünnter Salzsäure auf pH 1-2. Der Feststoff wird abfiltriert, mit Dichlormethan und Wasser gewaschen und getrocknet. Man erhält die Titelverbindung als Feststoff in einer HPLC-Reinheit von 88% (0,45g, 28% d. Th). Die UV-Absorption (kein Maximum bei 360 nm) belegt die O-Sulfonylierung des Produktes.
LC-MS: M+H = 423 (52%)
1 H-NMR (400 MHz, DMSO-D₆): δ (ppm) = 11.4 (s, breit, 1 H), 8.37 (d, 1 H), 7.31 (dt, 1H), 7.24 (dd, 1 H), 4.06 (s, 6H).

### Beispiel 11 (= Vergleichsbeispiel 11):

### Herstellung von 3-Phenyl-1 H-indol-2-yl-4-methylbenzolsulfonat

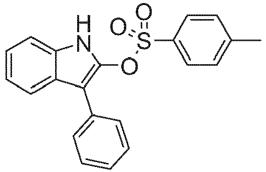

3-Phenyloxindol (1,15g), 4-Methylbenzolsulfonylchlorid (1,06g) und Natriumcarbonat (0,41g) werden in 5,5 ml Wasser und 11 ml Aceton vorgelegt und unter Rühren in einem vorgeheizten Ölbad auf 80°C erhitzt. Nach 20 Minuten wird 5 ml Aceton und 1 ml Wasser nachgegeben und weitere 30 Minuten erhitzt. Die heiße Lösung wird filtriert, der Feststoff mit 20 ml Wasser/Aceton (1:1) und Methanol gewaschen und im Vakuum getrocknet. Man erhält 0,59g Produkt in einer HPLC-Reinheit von 98% (29% d. Th). Eine Strukturaufklärung per NMR belegt, dass es sich um das O-sulfonylierte Produkt handelt. Aus dem Filtrat lassen sich noch 0,67g Produkt in einer HPLC-Reinheit von 40% isolieren (13% d. Th).
LC-MS: M-H = 362 (87%).
1 H-NMR (400 MHz, DMSO-D₆): δ (ppm) = 10.94 (s, 1 H), 7.90-7.95 (m, 2H), 7.83 (d, 1 H), 7.40-7.47 (m, 3H), 7.35 (t, 1 H), 7.22-7.28 (m, 4H), 7.20 (t, 1 H), 6.76 (d, 1 H), 2.33 (s, 3H).

### Beispiel 12:

### Herstellung von 1-[(Difluormethyl)sulfonyl]-3-(4,6-dimethoxy-1,3,5-triazin-2-yl)-7-fluor-3-methyl-1,3-dihydro-2H-indol-2-on

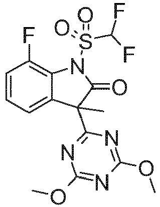

Schritt (a): 3-(4,6-Dimethoxy-1,3,5-triazin-2-yl)-7-fluor-3-methyl-1,3-dihydro-2H-indol-2-on 3-(4,6-Dimethoxy-1,3,5-triazin-2-yl)-7-fluor-1,3-dihydro-2H-indol-2-on (5,0g) wird in 225 ml Acetonitril vorgelegt, Natriumhydrid (0,73g, 60% in Mineralöl) zugegeben und nachgerührt, bis eine dicke Suspension entsteht. Nach Zugabe von lodmethan (7,0g) wird der Ansatz auf 60°C erwärmt und 11 Stunden bei 60°C gerührt. Das organische Lösungsmittel wird im Vakuum weitgehend abdestilliert. Zum teilweise kristallinen Rückstand wird 2-Propanol gegeben und das Produkt abfiltriert. Man erhält die Zwischenstufe als Feststoff in einer HPLC-Reinheit von 93% (3,46g, 64% d. Th). Die Struktur kann durch NMR-Spektroskopie bestätigt werden.
LC-MS: M+H = 305 (89%).
1 H-NMR (400 MHz, CDCl₃): δ (ppm) = 8.91 (s, 1 H), 6.95-7.03 (m, 2H), 6.91-6.95 (m, 1 H), 4.02 (s, 6H), 1.90 (s, 3H).

### Schritt (b): 1-[(Difluormethyl)sulfonyl]-3-(4,6-dimethoxy-1,3,5-triazin-2-yl)-7-fluor-3-methyl-1,3-dihydro-2H-indol-2-on

### Variante A:

3-(4,6-Dimethoxy-1,3,5-triazin-2-yl)-7-fluor-3-methyl-1,3-dihydro-2H-indol-2-on (0,50g) und 1-Methyl-1H-Imidazol (0,37g) werden in 5 ml Dichlormethan vorgelegt und auf -20°C gekühlt. Unter Rühren wird Difluormethansulfonylchlorid (0,58g) zugetropft und langsam auf Raumtemperatur erwärmt. Man rührt 3 Stunden bei Raumtemperatur. Durch HPLC-Analytik werden Edukt (67%), das gewünschte Produkt (25%) und weitere Komponenten detektiert. Der Ansatz wird auf 10ml Wasser gegeben, die Phasen getrennt und die organische Phase im Vakuum eingeengt. Der Rückstand wird durch Chromatographie aufgereinigt und das Produkt vom nicht umgesetzten Edukt abgetrennt. Man erhält die Titelverbindung in einer HPLC-Reinheit von 86% (0,16g, 22% d. Th). Mittels 2D-NMR kann die Struktur als N-sulfonylierte Verbindung bestätigt werden.
LC-MS: M+H = 419 (85%).
1H-NMR (400 MHz, CDCl₃): δ (ppm) = 7.10-7.25 (m, 3H), 6.67 (t, 1H), 4.02 (s, 6H), 1.94 (s, 3H).

### Variante B:

3-(4,6-Dimethoxy-1,3,5-triazin-2-yl)-7-fluor-3-methyl-1,3-dihydro-2H-indol-2-on (5,0g) wird in 40 ml Dichlormethan suspendiert. 1-Methyl-1 H-Imidazol (6,47g) und 1,4-Dimethylpiperazin (1,78g) werden hinzugegeben und die Lösung unter Stickstoff 30 Minuten bei 45°C gerührt. Man kühlt die Reaktionsmischung auf 0°C und gibt eine Lösung von Difluormethansulfonylchlorid (11,9g) in 20 ml Dichlormethan tropfenweise innerhalb 30 Minuten hinzu, wobei die Temperatur bei 0°C gehalten wird. Es wird eine Stunde bei 0°C nachgerührt. Nach Zugabe von 200 ml Dichlormethan wird die organische Phase mit 200 ml Salzsäure (2%) und mit Wasser gewaschen. Man gibt 100 ml Acetonitril und einige Tropfen N,N-Dimethylformamid zur organische Phase und wäscht mehrfach abwechselnd mit 200 ml-Portionen einer wässrigen Lösung von Kaliumhydroxid (3%) und mit Wasser. Das Lösungsmittel wird im Vakuum weitgehend abdestilliert. Der Rückstand wird in 50 ml Toluol gelöst und dreimal mit Wasser gewaschen. Das Lösungsmittel wird im Vakuum abdestilliert. Man erhält die Titelverbindung in einer HPLC-Reinheit von 93% (4,06g, 58% d. Th).

Die NMR-Signale des Produktes stimmen mit den Signalen des nach Variante A erhaltenen Produktes überein.

### Beispiel 13 (= Vergleichsbeispiel 13):

### Herstellung von 3-(4,6-Dimethoxy-1,3,5-triazin-2-yl)-7-fluor-1 H-indol-2-yl-4-methylbenzol-sulfonat

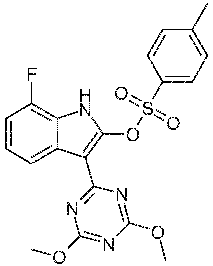

3-(4,6-Dimethoxy-1,3,5-triazin-2-yl)-7-fluor-1,3-dihydro-2H-indol-2-on (1,15g), 4-Methylbenzolsulfonylchlorid (1,06g) und Natriumcarbonat (0,41g) werden in 5,5 ml Wasser und 11 ml Aceton vorgelegt und unter Rühren in einem vorgeheitzten Ölbad auf 80°C erhitzt. Nach 45 Minuten wird die heiße Lösung filtriert, der Feststoff mit 20 ml Wasser/Aceton (1:1) und Methanol gewaschen und im Vakuum getrocknet. Man erhält 0,76g Produkt in einer HPLC-Reinheit von 97% (42% d. Th). Eine Strukturaufklärung per NMR belegt, dass es sich um das O-sulfonylierte Produkt handelt. Aus dem Filtrat lassen sich noch 0,41g Produkt in einer HPLC-Reinheit von 89% isolieren (21% d. Th).
LC-MS: M-H = 443 (95%).
1 H-NMR (400 MHz, DMSO-D₆): δ (ppm) = 13.33 (s, 1 H), 8.12 (d, 1 H), 7.64 (d, 2H), 7.30 (d, 2H), 7.10-7.22 (m, 2H), 3.97 (s, 6H), 2.33 (s, 3H).

### Beispiel 14:

### Herstellung von 7-Chlor-1-[(difluormethyl)sulfonyl]-3-(4,6-dimethoxy-1,3,5-triazin-2-yl)-1,3-dihydro-2H-indol-2-on

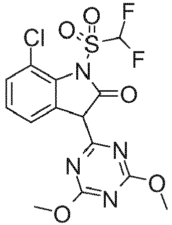

7-Chlor-3-(4,6-dimethoxy-1,3,5-triazin-2-yl)-1,3-dihydro-2H-indol-2-on (6,0g) wird in 150 ml Dichlormethan vorgelegt. 1-Methyl-1H-Imidazol (6,3g) und 1,4-Dimethylpiperazin (4,4g) wird zugegeben und die Reaktionsmischung eine Stunde bei Raumtemperatur gerührt. Man kühlt unter Stickstoff auf -60°C und tropft Difluormethansulfonylchlorid (14,4g) unter Rühren langsam zu, wobei die Temperatur unter -50°C gehalten wird. Der Ansatz wird auf -15°C erwärmt und zwei Tage bei dieser Temperatur unter zeitweiligem Rühren gehalten.
Zur Aufarbeitung wird 50 ml Wasser zugegeben und das organische Lösungsmittel im Vakuum weitgehend abdestilliert. Der Rückstand wird filtriert, mit Wasser gewaschen, in 100 ml 2-Propanol aufgenommen, eine Stunde nachgerührt, filtriert, mit 2-Propanol und Methanol gewaschen und getrocknet. Man erhält die Titelverbindung als Feststoff in einer HPLC-Reinheit von 99% (5,2g, 65% d. Th). Die UV-Absorption (Maximum bei 360 nm) sowie 2D-NMR-Spektren belegen die N-Sulfonylierung des Produktes.
LC-MS: M+H = 421 (100%).
1H-NMR (400 MHz, CDCl₃): δ (ppm) = 12.53 (s, 1H), 7.95 (dd, 1H), 7.13-7.21 (m, 2H), 6.96 (t, 1 H), 4.21 (s, 3H), 4.16 (s, 3H).

## Patentansprüche

1. Verfahren zur Herstellung von N-sulfonylsubstituierten Oxindolen der Formel (3), die in 3-Position einen 6-Ring-Heteroarylsubsitutenten (Q) aufweisen, 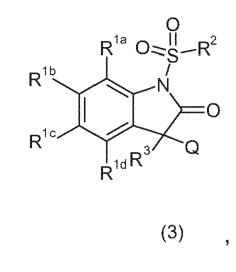 worin
R^{1a} bis R^{1d} unabhängig voneinander ausgewählt sind aus der Gruppe bestehend aus
Wasserstoff, Fluor, Chlor, Brom, lod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
(C₁-C₆)-Alkyl, wobei der Alkylrest unsubstituiert ist oder ganz oder teilweise mit Fluor substituiert ist, oder
(C₃-C₇)-Cycloalkyl, wobei der Cycloalkylrest unsubstituiert ist oder ganz oder teilweise mit Fluor substituiert ist,
steht,
R³ für
Wasserstoff, oder
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, steht, und
Q für
einen sechsgliedrigen heteroaromatischen Ring mit 1 bis 3 Stickstoffatomen steht, wobei der heteroaromatische Ring unsubstituiert ist oder durch einen oder mehrere Substituenten, ausgewählt aus der aus (C₁-C₄)-Alkyl, (C₁-C₄)-Alkoxy oder (C₃-C₇)-Cycloalkyl oder (C₃-C₇)-Cycloalkoxy oder (C₁-C₄)-Alkylthio bestehenden Gruppe, substituiert ist,
durch Umsetzung einer Oxindolverbindung der Formel (1) 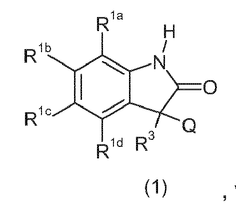 worin
R^{1a} bis R^{1d} sowie R³ und Q wie in Formel (3) definiert sind,
in einem Lösungsmittel mit einer Sulfonylverbindung (2) 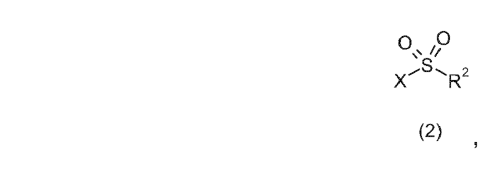 worin
R² wie in Formel (3) definiert ist, und
X als Abgangsgruppe für
Fluor, Chlor, Brom,1-Imidazolyl, 1 H-Benzotriazolyloxy, 1 H-Benzotriazolyl oder
O-SO2-R⁷, wobei R⁷ wie R² definiert ist und R² und R⁷ gleich oder verschieden sind, oder
N(R⁸)SO₂R⁹, wobei R⁸ für Carbonyl und R⁹ für unsubstituiertes oder substituiertes Phenyl stehen und R⁸ und R⁹ miteinander verbunden sind oder nicht verbunden sind,
steht,
wobei die Umsetzung in Gegenwart
- einer in 1-Position substituierten Imidazol-Base, oder
- eines Basen-Gemischs, das mindestens eine in 1-Position substituierte Imidazol-Base enthält,
durchgeführt wird.

2. Verfahren zur Herstellung von N-sulfonylsubstituierten 3-Triazinyloxindolen der Formel (3-1) nach Anspruch 1 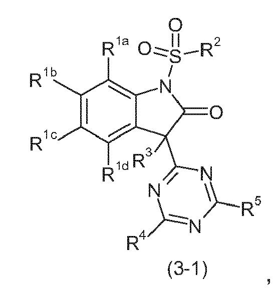 worin
R^{1a} bis R^{1d} und R³ wie in Anspruch 1 definiert sind, und
R² für (C₁-C₆)-Alkyl, oder (C₃-C₇)-Cycloalkyl steht, wobei der Alkylrest oder der Cycloalkylrest ganz oder teilweise mit Fluor substituiert ist, und
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
wobei Verbindungen der Formel (3-1), in denen
- R^{1a} für Fluor und
- R² für 2,2,-Difluorethyl oder 1,1,1-Trifluorethyl steht,
ausgenommen sind.

3. Verfahren nach Anspruch 1 oder 2, wobei R³ für Wasserstoff steht.

4. Verfahren nach einem der Ansprüche 1 bis 3, wobei R² für Difluormethan steht.

5. Verfahren nach einem der Ansprüche 1 bis 4, wobei die in 1-Position substituierten Imidazol-Basen ein
- 1-(C₁-C₆)-Alkyl-1H-Imidazol,
- 1-(C₁-C₇)-Cycloalkyl-1H-Imidazol,
- 1-Benzyl-1 H-Imidazol,
- 1-Aryl-1 H-Imidazol,
- 1-Hetaryl-1 H-Imidazol, oder
- ein Gemisch das mindestens zwei der genannten in 1-Position substituierten Imidazol-Basen enthält.

6. Verfahren nach Anspruch 5, wobei die in 1-Position substituierten Imidazol-Basen, die einzeln oder im Gemisch eingesetzt werden, 1-Methyl-1 H-Imidazol , 1-Butyl-1 H-Imidazol oder 1-Benzyl-1 H-Imidazol sind.

7. Verfahren nach einem der Ansprüche 1 bis 6, wobei das Basen-Gemisch mindestens eine in 1-Position substituierte Imidazol-Base enthält und zusätzlich mindestens eine
- tertiäre Aminbase der Formel (4) oder 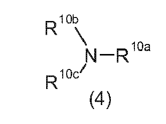
- substituierte Pyridinbase der Formel (5) oder 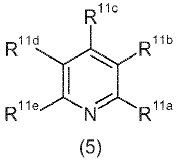
- ein Gemisch aus einer tertiären Aminbase der Formel (4) und einer substitiuerten Pyridinbase der Formel (5)
enthält.

8. Verfahren nach Anspruch 7, wobei die tertiären Aminbasen ausgewählt sind aus der Gruppe umfassend
- Diazabicyclo[2.2.2]octan (DABCO),
- 1-Methylmorpholin,
- 1-Ethylmorpholin
- N,N-Dimethylpiperazin und
- 1,2-Bis(dimethylamino)ethan.

9. Verfahren nach Anspruch 7, wobei als Pyridinbase 5-Ethyl-2-methylpyridin oder 3-Methylpyridin eingesetzt wird.

10. Verfahren nach einem der Ansprüche 1 bis 9, wobei die Umsetzung in den Lösungsmitteln
- Dichlormethan,
- 2-Methyltetrahydrofuran, oder
- Ethylacetat, oder
- in einem Gemisch der genannten Lösungsmitteln
erfolgt.

11. Verfahren nach einem der Ansprüche 1 bis 10, wobei die Zugabe der Reaktanden in einer Portion oder in mehreren Portionen über einen Zeitraum von 0,05 bis 6 Stunden erfolgt.

12. Verfahren nach einem der Ansprüche 1 bis 11, wobei die Reaktionstemperatur der Sulfonylierung im Bereich von -20°C bis +10°C liegt.

13. N-sulfonylsubstituierte 3-Triazinyloxindole der Formel (3-1) 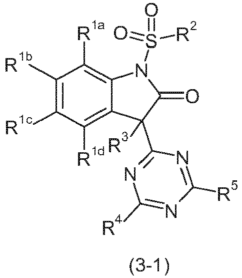 und deren Salze (3-1 a) 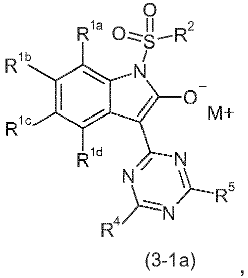 worin jeweils
R^{1a} bis R^{1d} unabhängig voneinander ausgewählt sind aus der Gruppe bestehend aus
Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
Methyl, wobei das Methyl ganz oder teilweise mit Fluor substituiert ist, oder (C₃-C₇)-Cycloalkyl, wobei der Cycloalkylrest ganz oder teilweise mit Fluor
substituiert ist,
steht,
R³ für
Wasserstoff, oder
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
steht,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
wobei in den Salzen der allgemeinen Formel (3-1a) M für Li, Na, K, Cs, Ba, Mg, Ca, Zn oder N(R^{c})₄, worin R^{c} = H oder C₁-C₆ Alkyl ist, steht und wobei sich die Anzahl der Gegenionen M⁺ nach der jeweiligen Ladung richtet, so dass eine insgesamt neutrale Verbindung der allgemeinen Formel (3-1a) entsteht.

14. N-sulfonylsubstituierten 3-Triazinyloxindolen der Formel (3-1) oder Salze der Formel (3-1 a) nach Anspruch 13, wobei
R^{1a} bis R^{1d} unabhängig voneinander ausgewählt sind aus der Gruppe bestehend aus
Wasserstoff, Fluor, Chlor sowie aus
(C₁-C₆)-Alkyl, wobei der Alkylrest verzweigt oder unverzweigt ist,
(C₁-C₆)-Alkoxy, wobei der Alkoxyrest verzweigt oder unverzweigt ist, und
R² für Difluormethyl oder Trifluormethyl steht,
R³ für Wasserstoff steht,
R⁴ und R⁵ unabhängig voneinander jeweils für
(C₁-C₄)-Alkyl, wobei der Alkylrest verzweigt oder unverzweigt ist,
(C₁-C₄)-Alkoxy, wobei der Alkoxyrest verzweigt oder unverzweigt ist,
stehen,
wobei in den Salzen der allgemeinen Formel (3-1a) M für Na und K steht.

15. N-sulfonylsubstituierten 3-Triazinyloxindolen der Formel (3-1) oder Salze der Formel (3-1 a) nach Anspruch 14, wobei
R^{1a} bis R^{1d} unabhängig voneinander ausgewählt sind aus der Gruppe bestehend aus Wasserstoff, Fluor, Chlor, Methoxy, und
R² für Difluormethyl steht,
R³ für Wasserstoff steht, und
R⁴ und R⁵ unabhängig voneinander jeweils für Methoxy stehen.

16. N-sulfonylsubstituierten 3-Triazinyloxindolen der Formel (3-1) oder Salze der Formel (3-1a) nach Anspruch 15 , wobei R^{1a} bis R^{1d} unabhängig voneinander ausgewählt sind aus der Gruppe bestehend aus Wasserstoff und Fluor.

17. Verwendung
- einer in 1-Position substituierten Imidazol-Base, oder
- eines Basen-Gemischs, das mindestens eine in 1-Position substituierte Imidazol-Base enthält,
zur Herstellung von N-sulfonylsubstituierten Oxindolen der Formel (3) gemäß einem der Ansprüche 1 bis 12 und zur Herstellung von N-sulfonylsubstituierten 3-Triazinyloxindolen der Formel (3-1) und deren Salze der Formel (3-1 a) nach einem der Ansprüche 13 bis 16.

18. Verwendung von Verbindungen der Formeln (3) hergestellt nach einem der Ansprüche 1 bis 12 und von Verbindungen der Formel (3-1) nach einem der Ansprüche 13 bis 16 sowie von deren Salzen (3-1 a) als Zwischenprodukte für die Herstellung von Feinchemikalien und Wirkstoffen der für die Landwirtschaft.

## Claims

1. A process for the preparation of N-sulfonyl-substituted oxindoles of the formula (3) which have a 6-ring heteroaryl substituent (Q) in the 3 position, 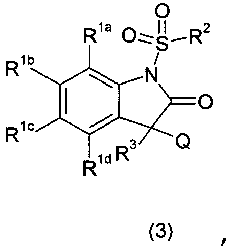 in which
R^{1a} to R^{1d}, independently of one another, are selected from the group consisting of
hydrogen, fluorine, chlorine, bromine, iodine, and also from
(C₁-C₆)-alkyl, where the alkyl radical is branched or unbranched and is unsubstituted or is substituted by one or more substituents selected from the group consisting of fluorine, chlorine, (C₁-C₄)-alkoxy or (C₃-C₇)-cycloalkyl,
(C₃-C₇)-cycloalkyl, where the cycloalkyl radical is unsubstituted or is substituted by one or more substituents selected from the group consisting of fluorine, chlorine, (C₁-C₄)-alkyl or (C₃-C₇)-cycloalkyl or (C₁-C₄)-alkoxy,
(C₁-C₆)-alkoxy, where the alkoxy radical is branched or unbranched and is unsubstituted or is substituted by one or more substituents selected from the group consisting of fluorine, chlorine, (C₁-C₄)-alkoxy or (C₃-C₇)-cycloalkyl,
(C₃-C₇)-cycloalkoxy, where the cycloalkoxy radical is unsubstituted or is substituted by one or more substituents selected from the group consisting of fluorine, chlorine, (C₁-C₄)-alkyl or (C₁-C₄)-alkoxy,
(C₁-C₆)-alkylthio, where the alkylthio radical is branched or unbranched and is unsubstituted or is substituted by one or more substituents selected from the group consisting of fluorine, chlorine, (C₁-C₄)-alkyl or (C₁-C₄)-alkoxy,
(C₃-C₇)-cycloalkylthio, where the cycloalkylthio radical is unsubstituted or is substituted by one or more substituents selected from the group consisting of fluorine, chlorine, (C₁-C₄)-alkyl or (C₁-C₄)-alkoxy, and phenyl or 1-naphthyl or 2-naphthyl or a five- or six-membered heteroaromatic ring having 1 to 2 heteroatoms, where the heteroatoms are selected, independently of one another, from the group consisting of O or N and where the aryl or heteroaryl radical is unsubstituted or is substituted by one or more substituents selected from the group consisting of fluorine, chlorine, bromine, iodine, (C₁-C₄)-alkyl, (C₁-C₄)-alkoxy or (C₃-C₇)-cycloalkyl or (C₁-C₄)-alkylthio, and
R² is
(C₁-C₆)-alkyl, where the alkyl radical is unsubstituted or is completely or partially substituted with fluorine, or
(C₃-C₇)-cycloalkyl, where the cycloalkyl radical is unsubstituted or is completely or partially substituted with fluorine,
R³ is
hydrogen or
(C₁-C₆)-alkyl, where the alkyl radical is branched or unbranched and is unsubstituted or is substituted by one or more substituents selected from the group consisting of fluorine, chlorine, (C₁-C₄)-alkoxy or (C₃-C₇)-cycloalkyl,
(C₁-C₆)-alkylthio, where the alkylthio radical is unsubstituted or is substituted by one or more substituents selected from the group consisting of fluorine, chlorine, (C₁-C₄)-alkyl or (C₁-C₄)-alkoxy,
and
Q is a six-membered heteroaromatic ring having 1 to 3 nitrogen atoms, where the heteroaromatic ring is unsubstituted or is substituted by one or more substituents selected from the group consisting of (C₁-C₄)-alkyl, (C₁-C₄)-alkoxy or (C₃-C₇)-cycloalkyl or (C₃-C₇)-cycloalkoxy or (C₁-C₄)-alkylthio,
by reaction of an oxindole compound of the formula (1) 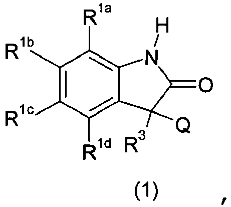 in which
R^{1a} to R^{1d}, and R³ and Q are as defined in formula (3),
in a solvent with a sulfonyl compound (2) 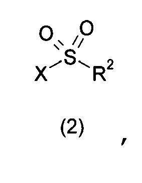 in which
R² is as defined in formula (3), and
X, as leaving group, is
fluorine, chlorine, bromine, 1-imidazolyl, 1H-benzotriazolyloxy, 1H-benzotriazolyl or O-SO2-R⁷, where R⁷ is defined as R² and R² and R⁷ are identical or different, or
N(R⁸)SO₂R⁹, where R⁸ is carbonyl and R⁹ is unsubstituted or substituted phenyl, and R⁸ and
R⁹ are bonded with one another or are not bonded,
where the reaction is carried out in the presence of
- an imidazole base substituted in the 1 position, or
- a base mixture which comprises at least one imidazole base substituted in the 1 position.

2. The process for the preparation of N-sulfonyl-substituted 3-triazinyloxindoles of the formula (3-1) as claimed in claim 1 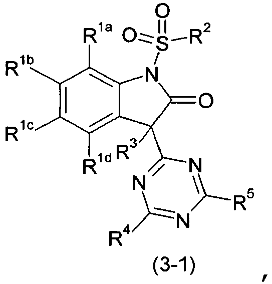 in which
R^{1a} to R^{1d} and R³ are as defined in claim 1, and
R² is (C₁-C₆)-alkyl, or (C₃-C₇)-cycloalkyl, where the alkyl radical or the cycloalkyl radical is completely or partially substituted with fluorine, and
R⁴ and R⁵, independently of one another, are in each case
hydrogen,
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl,
(C₁-C₆)-alkoxy, where the alkoxy radical is branched or unbranched and is unsubstituted or is substituted by one or more substituents selected from the group consisting of fluorine, chlorine, (C₁-C₄)-alkoxy or (C₃-C₇)-cycloalkyl,
where compounds of the formula (3-1), in which
- R^{1a} is fluorine and
- R² is 2,2,-difluoroethyl or 1,1,1-trifluoroethyl, are excluded.

3. The process as claimed in claim 1 or 2, where R³ is hydrogen.

4. The process as claimed in one of claims 1 to 3, where R² is difluoromethane.

5. The process as claimed in one of claims 1 to 4, where the imidazole bases substituted in the 1 position contains a
- 1-(C₁-C₆)-alkyl-1H-imidazole,
- 1-(C₁-C₇)-cycloalkyl-1H-imidazole,
- 1-benzyl-1H-imidazole,
- 1-aryl-1H-imidazole,
- 1-hetaryl-1H-imidazole, or
- a mixture which comprises at least two of the specified imidazole bases substituted in the 1 position.

6. The process as claimed in claim 5, where the imidazole bases substituted in the 1 position, which are used individually or in a mixture, are 1-methyl-1H-imidazole, 1-butyl-1H-imidazole or 1-benzyl-1H-imidazole.

7. The process as claimed in one of claims 1 to 6, where the base mixture comprises at least one imidazole base substituted in the 1 position and additionally comprises at least one
- tertiary amine base of the formula (4) or 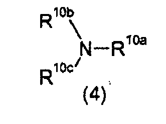
- substituted pyridine base of the formula (5) or 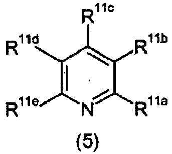
- a mixture of a tertiary amine base of the formula (4) and a substituted pyridine base of the formula (5).

8. The process as claimed in claim 7, where the tertiary amine bases are selected from the group comprising
- diazabicyclo[2.2.2]octane (DABCO),
- 1-methylmorpholine,
- 1-ethylmorpholine
- N,N-dimethylpiperazine and
- 1,2-bis(dimethylamino)ethane.

9. The process as claimed in claim 7, where the pyridine base used is 5-ethyl-2-methylpyridine or 3-methylpyridine.

10. The process as claimed in one of claims 1 to 9, where the reaction takes place in the solvents
- dichloromethane,
- 2-methyltetrahydrofuran, or
- ethyl acetate, or
- in a mixture of the specified solvents.

11. The process as claimed in one of claims 1 to 10, where the addition of the reactants takes place in one portion or in two or more portions over a period of from 0.05 to 6 hours.

12. The process as claimed in one of claims 1 to 11, where the reaction temperature of the sulfonylation is in the range from -20°C to +10°C.

13. An N-sulfonyl-substituted 3-triazinyloxindole of the formula (3-1) 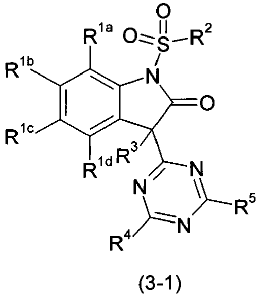 or a salt thereof (3-1a) 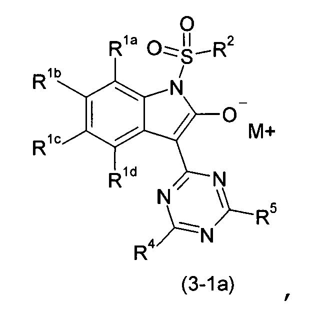 in which, in each case,
R^{1a} to R^{1d}, independently of one another, are selected from the group consisting of
hydrogen, fluorine, chlorine, bromine, iodine, and also of
(C₁-C₆)-alkyl, where the alkyl radical is branched or unbranched and is unsubstituted or is substituted by one or more substituents selected from the group consisting of fluorine, chlorine, (C₁-C₄)-alkoxy or (C₃-C₇)-cycloalkyl,
(C₃-C₇)-cycloalkyl, where the cycloalkyl radical is unsubstituted or is substituted by one or more substituents selected from the group consisting of fluorine, chlorine, (C₁-C₄)-alkyl or (C₃-C₇)-cycloalkyl or (C₁-C₄)-alkoxy,
(C₁-C₆)-alkoxy, where the alkoxy radical is branched or unbranched and is unsubstituted or is substituted by one or more substituents selected from the group consisting of fluorine, chlorine, (C₁-C₄)-alkoxy or (C₃-C₇)-cycloalkyl,
(C₃-C₇)-cycloalkoxy, where the cycloalkoxy radical is unsubstituted or is substituted by one or more substituents selected from the group consisting of fluorine, chlorine, (C₁-C₄)-alkyl or (C₁-C₄)-alkoxy,
(C₁-C₆)-alkylthio, where the alkylthio radical is branched or unbranched and is unsubstituted or is substituted by one or more substituents selected from the group consisting of fluorine, chlorine, (C₁-C₄)-alkyl or (C₁-C₄)-alkoxy,
(C₃-C₇)-cycloalkylthio, where the cycloalkylthio radical is unsubstituted or is substituted by one or more substituents selected from the group consisting of fluorine, chlorine, (C₁-C₄)-alkyl or (C₁-C₄)-alkoxy, and
phenyl or 1-naphthyl or 2-naphthyl or a five- or six-membered heteroaromatic ring having 1 to 2 heteroatoms, where the heteroatoms, independently of one another, are selected from the group consisting of O or N, and where the aryl or heteroaryl radical is unsubstituted or is substituted by one or more substituents selected from the group consisting of fluorine, chlorine, bromine, iodine, (C₁-C₄)-alkyl, (C₁-C₄)-alkoxy or (C₃-C₇)-cycloalkyl or (C₁-C₄) -alkylthio, and
R² is
methyl, where the methyl is completely or partially substituted with fluorine, or
(C₃-C₇)-cycloalkyl, where the cycloalkyl radical is completely or partially substituted with fluorine,
R³ is
hydrogen, or
(C₁-C₆)-alkyl, where the alkyl radical is branched or unbranched and is unsubstituted or is substituted by one or more substituents selected from the group consisting of fluorine, chlorine, (C₁-C₄)-alkoxy or (C₃-C₇)-cycloalkyl,
(C₁-C₆)-alkylthio, where the alkylthio radical is unsubstituted or is substituted by one or more substituents selected from the group consisting of fluorine, chlorine, (C₁-C₄)-alkyl or (C₁-C₄)-alkoxy,
R⁴ and R⁵, independently of one another, are in each case
hydrogen,
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl,
(C₁-C₆)-alkoxy, where the alkoxy radical is branched or unbranched and is unsubstituted or is substituted by one or more substituents selected from the group consisting of fluorine, chlorine, (C₁-C₄)-alkoxy or (C₃-C₇)-cycloalkyl,
where, in the salt of the formula (3-1a), M is Li, Na, K, Cs, Ba, Mg, Ca, Zn or N(R^{c})₄, where R^{c} = H or C₁-C₆ alkyl, and where the number of counterions M⁺ is determined by the particular charge, such that an overall neutral compound of the formula (3-1a) is formed.

14. The N-sulfonyl-substituted 3-triazinyloxindole of the formula (3-1) or a salt of the formula (3-1a) as claimed in claim 13, where
R^{1a} to R^{1d}, independently of one another, are selected from the group consisting of
hydrogen, fluorine, chlorine, and also from
(C₁-C₆)-alkyl, where the alkyl radical is branched or unbranched,
(C₁-C₆)-alkoxy, where the alkoxy radical is branched or unbranched, and
R² is difluoromethyl or trifluoromethyl,
R³ is hydrogen,
R⁴ and R⁵, independently of one another, are in each case
(C₁-C₄)-alkyl, where the alkyl radical is branched or unbranched,
(C₁-C₄)-alkoxy, where the alkoxy radical is branched or unbranched,
where, in the salt of the formula (3-1a), M is Na and K.

15. The N-sulfonyl-substituted 3-triazinyloxindole of the formula (3-1) or a salt of the formula (3-1 a) as claimed in claim 14, where
R^{1a} to R^{1d}, independently of one another, are selected from the group consisting of hydrogen, fluorine, chlorine, methoxy, and
R² is difluoromethyl,
R³ is hydrogen, and
R⁴ and R⁵, independently of one another, are in each case methoxy.

16. The N-sulfonyl-substituted 3-triazinyloxindole of the formula (3-1) or a salt of the formula (3-1a) as claimed in claim 15, where R^{1a} to R^{1d}, independently of one another, are selected from the group consisting of hydrogen and fluorine.

17. The use of
- an imidazole base substituted in the 1 position, or
- a base mixture which comprises at least one imidazole base substituted in the 1 position,
for the preparation of N-sulfonyl-substituted oxindoles of the formula (3) as claimed in one of claims 1 to 12 and for the preparation of N-sulfonyl-substituted 3-triazinyloxindoles of the formula (3-1) and salts thereof of the formula (3-1a) as claimed in one of claims 13 to 16.

18. The use of compounds of the formulae (3) prepared as claimed in one of claims 1 to 12 and of compounds of the formula (3-1) as claimed in one of claims 13 to 16, and also of salts thereof (3-1a) as intermediates for producing fine chemicals and active ingredients of the for agriculture.

## Revendications

1. Procédé de fabrication d'oxindoles à substitution N-sulfonyle de formule (3), qui comprennent en position 3 un substituent hétéroaryle à cycle à 6 éléments (Q), 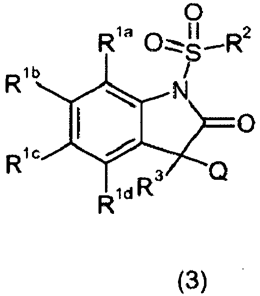 dans laquelle
R^{1a} à R^{1d} sont choisis indépendamment les uns des autres dans le groupe constitué par
hydrogène, fluor, chlore, brome, iode, ainsi que alkyle en (C₁-C₆), le radical alkyle étant ramifié ou non ramifié, et non substitué ou substitué par un ou plusieurs substituants, choisis dans le groupe constitué par fluor, chlore, alcoxy en (C₁-C₄) ou cycloalkyle en (C₃-C₇),
cycloalkyle en (C₃-C₇), le radical cycloalkyle étant non substitué ou substitué par un ou plusieurs substituants choisis dans le groupe constitué par fluor, chlore, alkyle en (C₁-C₄) ou cycloalkyle en (C₃-C₇) ou alcoxy en (C₁-C₄),
alcoxy en (C₁-C₆), le radical alcoxy étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
cycloalcoxy en (C₃-C₇), le radical cycloalcoxy étant non substitué ou substitué par un ou plusieurs substituants choisis dans le groupe constitué par fluor, chlore, alkyle en (C₁-C₄) ou alcoxy en (C₁-C₄),
alkylthio en (C₁-C₆), le radical alkylthio étant ramifié ou non ramifié, et non substitué ou substitué par un ou plusieurs substituants choisis dans le groupe constitué par fluor, chlore, alkyle en (C₁-C₄) ou alcoxy en (C₁-C₄),
cycloalkylthio en (C₃-C₇), le radical cycloalkylthio étant non substitué ou substitué par un ou plusieurs substituants choisis dans le groupe constitué par fluor, chlore, alkyle en (C₁-C₄) ou alcoxy en (C₁-C₄), et phényle ou 1-naphtyle ou 2-naphtyle ou un cycle hétéroaromatique à cinq ou six éléments contenant 1 à 2 hétéroatomes, les hétéroatomes étant choisis indépendamment l'un de l'autre dans le groupe constitué par O et N, et le radical aryle ou hétéroaryle étant non substitué ou substitué par un ou plusieurs substituants choisis dans le groupe constitué par fluor, chlore, brome, iode, alkyle en (C₁-C₄), alcoxy en (C₁-C₄) ou cycloalkyle en (C₃-C₇) ou alkylthio en (C₁-C₄), et
R² représente
alkyle en (C₁-C₆), le radical alkyle étant non substitué ou substitué en totalité ou en partie avec fluor, ou cycloalkyle en (C₃-C₇), le radical cycloalkyle étant non substitué ou substitué en totalité ou en partie avec fluor,
R³ représente
hydrogène ou
alkyle en (C₁-C₆), le radical alkyle étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
alkylthio en (C₁-C₆), le radical alkylthio étant non substitué ou substitué par un ou plusieurs substituants choisis dans le groupe constitué par fluor, chlore, alkyle en (C₁-C₄) ou alcoxy en (C₁-C₄),
et
Q représente
un cycle hétéroaromatique à six éléments contenant 1 à 3 atomes d'azote, le cycle hétéroaromatique étant non substitué ou substitué par un ou plusieurs substituants choisis dans le groupe constitué par alkyle en (C₁-C₄), alcoxy en (C₁-C₄) ou cycloalkyle en (C₃-C₇) ou cycloalcoxy en (C₃-C₇) ou alkylthio en (C₁-C₄), par mise en réaction d'un composé d'oxindole de formule (1) 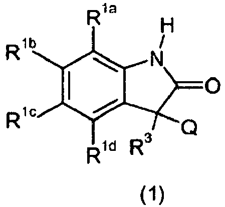 dans laquelle
R^{1a} à R^{1d}, ainsi que R³ et Q sont tels que définis dans la formule (3),
dans un solvant avec un composé de sulfonyle (2) 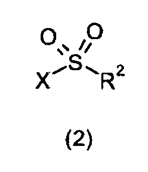 dans lequel
R² est tel que défini dans la formule (3), et
X représente en tant que groupe partant fluor, chlore, brome, 1-imidazolyle, 1H-benzotriazolyloxy, 1H-benzotriazolyle ou
O-SO₂-R⁷, R⁷ étant défini tel que R², et R² et R⁷ étant identiques ou différents, ou
N(R⁸)SO₂R⁹, R⁸ représentant carbonyle et R⁹ représentant phényle non substitué ou substitué, et R⁸ et R⁹ étant reliés l'un à l'autre ou non reliés l'un à l'autre,
la réaction étant réalisée en présence
- d'une base imidazole substituée en position 1, ou
- d'un mélange de bases qui contient au moins une base imidazole substituée en position 1.

2. Procédé de fabrication de 3-triazinyloxindoles à substitution N-sulfonyle de formule (3-1) selon la revendication 1 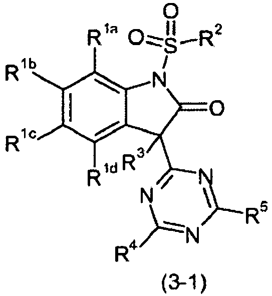 dans laquelle
R^{1a} à R^{1d} et R³ sont tels que définis dans la revendication 1,
R² représente alkyle en (C₁-C₆) ou cycloalkyle en (C₃-C₇), le radical alkyle ou le radical cycloalkyle étant substitué en totalité ou en partie avec fluor, et
R⁴ et R⁵ représentent chacun indépendamment l'un de l'autre
hydrogène,
alkyle en (C₁-C₆), le radical alkyle étant non substitué ou substitué par un ou plusieurs substituants choisis dans le groupe constitué par fluor, chlore, alcoxy en (C₁-C₄) ou cycloalkyle en (C₃-C₇),
alcoxy en (C₁-C₆), le radical alcoxy étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
les composés de formule (3-1) dans lesquels
- R^{1a} représente fluor et
- R² représente 2,2-difluoroéthyle ou 1,1,1-trifluoroéthyle,
étant exclus.

3. Procédé selon la revendication 1 ou 2, dans lequel R³ représente hydrogène.

4. Procédé selon l'une quelconque des revendications 1 à 3, dans lequel R² représente difluorométhane.

5. Procédé selon l'une quelconque des revendications 1 à 4, dans lequel les bases imidazole substituées en position 1 contient
- un 1-alkyle en (C₁-C₆)-1H-imidazole,
- un 1-cycloalkyle en (C₁-C₇)-1H-imidazole,
- un 1-benzyl-1H-imidazole,
- un 1-aryl-1H-imidazole,
- un 1-hétaryl-1H-imidazole, ou
- un mélange qui contient au moins deux des bases imidazole substituées en position 1 citées.

6. Procédé selon la revendication 5, dans laquelle les bases imidazole substituées en position 1, qui sont utilisées individuellement ou en mélange, sont le 1-méthyl-1H-imidazole, le 1-butyl-1H-imidazole ou le 1-benzyl-1H-imidazole.

7. Procédé selon l'une quelconque des revendications 1 à 6, dans lequel le mélange de bases contient au moins une base imidazole substituée en position 1 et en outre au moins
- une base amine tertiaire de formule (4) 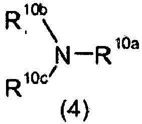 ou
- une base pyridine substituée de formule (5) 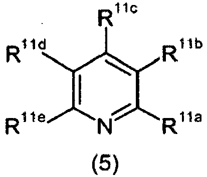 ou
- un mélange d'une base amine tertiaire de formule (4) et d'une base pyridine substituée de formule (5).

8. Procédé selon la revendication 7, dans lequel les bases amine tertiaires sont choisies dans le groupe comprenant
- le diazabicyclo[2.2.2]octane (DABCO),
- la 1-méthylmorpholine,
- la 1-éthylmorpholine,
- la N,N-diméthylpipérazine et
- le 1,2-bis(diméthylamino)éthane.

9. Procédé selon la revendication 7, dans lequel la 5-éthyl-2-méthylpyridine ou la 3-méthylpyridine est utilisée en tant que base pyridine.

10. Procédé selon l'une quelconque des revendications 1 à 9, dans lequel la réaction a lieu dans les solvants
- dichlorométhane,
- 2-méthyltétrahydrofurane ou
- acétate d'éthyle ou
- dans un mélange des solvants cités.

11. Procédé selon l'une quelconque des revendications 1 à 10, dans lequel l'ajout des réactifs a lieu en une partie ou en plusieurs parties en une période de temps de 0,05 à 6 heures.

12. Procédé selon l'une quelconque des revendications 1 à 11, dans lequel la température de réaction de la sulfonylation se situe dans la plage allant de -20 °C à +10 °C.

13. 3-Triazinyloxindoles à substitution N-sulfonyle de formule (3-1) 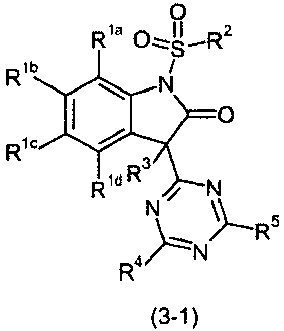 et leurs sels (3-1a) 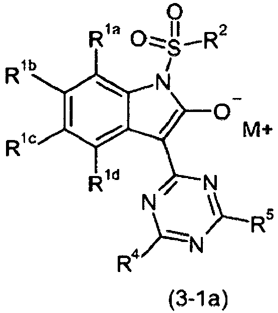 dans lesquels
R^{1a} à R^{1d} sont choisis indépendamment les uns des autres dans le groupe constitué par
hydrogène, fluor, chlore, brome, iode, ainsi que alkyle en (C₁-C₆), le radical alkyle étant ramifié ou non ramifié, et non substitué ou substitué par un ou plusieurs substituants, choisis dans le groupe constitué par fluor, chlore, alcoxy en (C₁-C₄) ou cycloalkyle en (C₃-C₇),
cycloalkyle en (C₃-C₇), le radical cycloalkyle étant non substitué ou substitué par un ou plusieurs substituants choisis dans le groupe constitué par fluor, chlore, alkyle en (C₁-C₄) ou cycloalkyle en (C₃-C₇) ou alcoxy en (C₁-C₄),
alcoxy en (C₁-C₆), le radical alcoxy étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
cycloalcoxy en (C₃-C₇), le radical cycloalcoxy étant non substitué ou substitué par un ou plusieurs substituants choisis dans le groupe constitué par fluor, chlore, alkyle en (C₁-C₄) ou alcoxy en (C₁-C₄),
alkylthio en (C₁-C₆), le radical alkylthio étant ramifié ou non ramifié, et non substitué ou substitué par un ou plusieurs substituants choisis dans le groupe constitué par fluor, chlore, alkyle en (C₁-C₄) ou alcoxy en (C₁-C₄),
cycloalkylthio en (C₃-C₇), le radical cycloalkylthio étant non substitué ou substitué par un ou plusieurs substituants choisis dans le groupe constitué par fluor, chlore, alkyle en (C₁-C₄) ou alcoxy en (C₁-C₄), et phényle ou 1-naphtyle ou 2-naphtyle ou un cycle hétéroaromatique à cinq ou six éléments contenant 1 à 2 hétéroatomes, les hétéroatomes étant choisis indépendamment l'un de l'autre dans le groupe constitué par O et N, et le radical aryle ou hétéroaryle étant non substitué ou substitué par un ou plusieurs substituants choisis dans le groupe constitué par fluor, chlore, brome, iode, alkyle en (C₁-C₄), alcoxy en (C₁-C₄) ou cycloalkyle en (C₃-C₇) ou alkylthio en (C₁-C₄), et
R² représente
méthyle, le méthyle étant substitué en totalité ou en partie avec fluor, ou
cycloalkyle en (C₃-C₇), le radical cycloalkyle étant substitué en totalité ou en partie avec fluor,
R³ représente
hydrogène ou
alkyle en (C₁-C₆), le radical alkyle étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
alkylthio en (C₁-C₆), le radical alkylthio étant non substitué ou substitué par un ou plusieurs substituants choisis dans le groupe constitué par fluor, chlore, alkyle en (C₁-C₄) ou alcoxy en (C₁-C₄),
R⁴ et R⁵ représentent chacun indépendamment l'un de l'autre
hydrogène,
alkyle en (C₁-C₆), le radical alkyle étant non substitué ou substitué par un ou plusieurs substituants choisis dans le groupe constitué par fluor, chlore, alcoxy en (C₁-C₄) ou cycloalkyle en (C₃-C₇),
alcoxy en (C₁-C₆), le radical alcoxy étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
dans les sels de formule générale (3-1a), M représentant Li, Na, K, Cs, Ba, Mg, Ca, Zn ou N(R^{c})₄, avec R^{c} = H ou alkyle en C₁-C₆, et le nombre de contre-ions M⁺ étant déterminé par la charge respective, de manière à former un composé de formule générale (3-1a) au total neutre.

14. 3-Triazinyloxindoles à substitution N-sulfonyle de formule (3-1) ou sels de formule (3-1a) selon la revendication 13, dans lesquels
R^{1a} à R^{1d} sont choisis indépendamment les uns des autres dans le groupe constitué par
hydrogène, fluor, chlore, ainsi que
alkyle en (C₁-C₆), le radical alkyle étant ramifié ou non ramifié,
alcoxy en (C₁-C₆), le radical alcoxy étant ramifié ou non ramifié, et
R² représente difluorométhyle ou trifluorométhyle,
R³ représente hydrogène,
R⁴ et R⁵ représentent chacun indépendamment l'un de l'autre
alkyle en (C₁-C₄), le radical alkyle étant ramifié ou non ramifié,
alcoxy en (C₁-C₄), le radical alcoxy étant ramifié ou non ramifié,
dans les sels de formule générale (3-1a), M représentant Na et K.

15. 3-Triazinyloxindoles à substitution N-sulfonyle de formule (3-1) ou sels de formule (3-1a) selon la revendication 14, dans lesquels
R^{1a} à R^{1d} sont choisis indépendamment les uns des autres dans le groupe constitué par hydrogène, fluor, chlore, méthoxy, et
R² représente difluorométhyle,
R³ représente hydrogène, et
R⁴ et R⁵ représentent chacun indépendamment l'un de l'autre méthoxy.

16. 3-Triazinyloxindoles à substitution N-sulfonyle de formule (3-1) ou sels de formule (3-1a) selon la revendication 15, dans lesquels R^{1a} à R^{1d} sont choisis indépendamment les uns des autres dans le groupe constitué par hydrogène et fluor.

17. Utilisation
- d'une base imidazole substituée en position 1 ou
- d'un mélange de bases qui contient au moins une base imidazole substituée en position 1,
pour la fabrication d'oxindoles à substitution N-sulfonyle de formule (3) selon l'une quelconque des revendications 1 à 12, et pour la fabrication de 3-triazinyloxindoles à substitution N-sulfonyle de formule (3-1) et leurs sels de formule (3-1a) selon l'une quelconque des revendications 13 à 16.

18. Utilisation de composés de formule (3) fabriqués selon l'une quelconque des revendications 1 à 12 et de composés de formule (3-1) selon l'une quelconque des revendications 13 à 16, ainsi que de leurs sels (3-1a) en tant que produits intermédiaires pour la fabrication de produits chimiques fins et d'agents actifs du pour l'agriculture.
